# EUROPEAN PATENT SPECIFICATION

(11) **EP 3 411 378 B1**
(45) Date of publication and mention of the grant of the patent: **25.03.2020**
(21) Application number: 17702119.3
(22) Date of filing: 01.02.2017
(51) Int. Cl.: C07F 9/6561, A61K 31/675, A61P 31/18

(54) **CRYSTALLINE FORMS OF TENOFOVIR ALAFENAMIDE MONOFUMARATE**
KRISTALLINE FORM VON TENOFOVIR ALAFENAMIDE MONOFUMARAT
FORME CRISTALLINE DU MONOFUMARATE DE TÉNOFOVIR ALAFÉNAMIDE

(30) Priority: 02.02.2016 EP 16153952; 19.04.2016 EP 16166063
(43) Date of publication of application: 12.12.2018
(73) Proprietor: Sandoz AG, 4056 Basel (CH)
(72) Inventor: HOTTER, Andreas, 6250 Kundl (AT); LENGAUER, Hannes, 6250 Kundl (AT)
(74) Representative: Kluschanzoff, Harald
(86) International application number: PCT/EP2017/052121
(87) International publication number: WO 2017/134089

(56) References cited:
- EP-A1- 2 891 658
- WO-A1-2013/025788
- WO-A2-02/08241
- WO-A2-2015/040640
- WO-A2-2015/107451
- CN-A- 104 558 036
- CN-A- 105 237 571

## Description

### FIELD OF THE INVENTION

The present invention relates to crystalline forms of tenofovir alafenamide monofumarate and methods for their preparation. Furthermore, the invention relates to the use of one or more of the crystalline forms of tenofovir alafenamide monofumarate of the present invention for the preparation of pharmaceutical compositions as well as to pharmaceutical compositions comprising an effective amount of one or more of said forms. The pharmaceutical compositions of the present invention can be used as medicaments, in particular for the treatment and/or prophylaxis of viral infections such as HIV infections.

### BACKGROUND OF THE INVENTION

Tenofovir alafenamide, a prodrug of tenofovir, is chemically designated as 9-[(*R*)-2-[[(*S*)-[[(*S*)-1-(isopropoxycarbonyl)ethyl]amino]phenoxyphosphinyl]methoxy]propyl]adenine and can be represented by the following chemical structure according to formula (I):

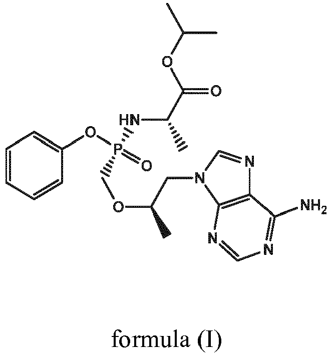

Tenofovir alafenamide is an antiviral compound useful for the treatment and/or prophylaxis of viral infections including infections caused by DNA viruses, RNA viruses, herpesviruses (e.g. CMV, HSV 1, HSV 2, VZV), retroviruses, hepadnaviruses (e.g. HBV), papillomavirus, hantavirus, adenoviruses and HIV. Tenofovir alafenamide may be administered alone or in combination with other antiviral agents to patients in need.

WO 02/008241 A2 discloses tenofovir alafenamide and a tenofovir alafenamide monofumarate form as well as processes for their preparations. Another disclosure of a physical form of tenofovir alafenamide monofumarate can be found in Example 6 and Figure 2 of WO 2015/040640 A2. According to the latter document, the process for the preparation is similar to the process described in Example 4 of WO 02/008241 A2.

WO 2015/107451 A2 and CN 105237571 A describe reactions of tenofovir alafenamide with fumaric acid in acetonitrile and ethanol or methanol respectively.

WO 2013/025788 A1 describes a crystalline hemifumarate form of tenofovir alafenamide. The document further discloses in paragraph [0071] that the solid form of tenofovir alafenamide monofumarate obtained by the procedure described in WO 02/084241 A2 is thermodynamically unstable.

Different solid state forms of an active pharmaceutical ingredient often possess different properties. Different properties of solid state forms can allow for improved formulations, for example, formulations with improved dissolution profile, or with improved stability and shelf-life. Also processing or handling of the active pharmaceutical ingredient during the formulation process may be improved. Thus, new solid state forms of an active pharmaceutical ingredient can have desirable processing properties. They can be easier to handle, better suited for storage, and/or allow for better purification, compared to previously known solid state forms.

The tendency of a drug substance to absorb water from the environment can affect the pharmaceutical behavior and quality of a drug product. Water absorption for example can lead to chemical degradation (e.g. via hydrolysis), trigger changes of the physical form (e.g. via hydrate formation), lead to changes in dissolution behavior and influence powder properties such as flowability, compactability, tableting and compression behavior etc. That's why, non-hygroscopic substances are preferably used, as no precautious measures need to be taken, which renders pharmaceutical processes cheaper and more reliable. Thus, there is a strong need for the provision of a non-hygroscopic form of tenofovir alafenamide monofumarate.

Furthermore, the sudden appearance or disappearance of a polymorph can present a problem in process development. Similarly, serious pharmaceutical consequences arise if transformation occurs in a dosage form. It is therefore desired to use a thermodynamically stable solid state form of an active pharmaceutical ingredient. Hence, there is also a strong need for the provision of a solid state form of tenofovir alafenamide monofumarate which is thermodynamically stable.

### SUMMARY OF THE INVENTION

The present invention provides a crystalline form of tenofovir alafenamide monofumarate, herein also designated "form II". In particular, the present invention relates to crystalline form II of tenofovir alafenamide monofumarate characterized by having an X-ray powder diffraction (XRPD) pattern [powder X-ray diffractogram - PXRD] comprising reflections at 2-theta angles of (7.3 ± 0.2)°, (9.4 ± 0.1)° and (10.1 ± 0.1)°.

In addition, the present invention relates to a crystalline form of tenofovir alafenamide monofumarate, herein also designated "form III". In particular, the present invention relates to crystalline form III of tenofovir alafenamide monofumarate characterized by having an X-ray powder diffraction (XRPD) pattern [powder X-ray diffractogram - PXRD] comprising reflections at 2-theta angles of (5.6 ± 0.1)°, (7.3 ± 0.2)° and (10.5 ± 0.1)°.

Furthermore, the present invention relates to a crystalline solvate of tenofovir alafenamide monofumarate, herein also designated "form S". In particular, the present invention relates to crystalline form S of tenofovir alafenamide monofumarate characterized by having an X-ray powder diffraction (XRPD) pattern [powder X-ray diffractogram - PXRD] comprising reflections at 2-theta angles of (4.8 ± 0.2)°, (10.3 ± 0.2)° and (10.6 ± 0.2)°. The invention further relates to a composition comprising one or more of the aforementioned crystalline form(s) of tenofovir alafenamide monofumarate and processes for the preparation of the crystalline form(s) and the composition. In addition, the invention relates to the use of the crystalline form(s) II and/or III of tenofovir alafenamide monofumarate of the present invention for the preparation of a pharmaceutical composition and to a pharmaceutical composition comprising an effective amount of the crystalline form(s) II and/or III of tenofovir alafenamide monofumarate of the present invention.

### Abbreviations

- PXRD: powder X-ray diffractogram
- XRPD: X-ray powder diffraction
- DSC: differential scanning calorimetry
- TGA: thermogravimetric analysis
- GMS: gravimetric moisture sorption
- THF: tetrahydrofuran
- MTBE: methyl tert-butyl ether
- RH: relative humidity
- MF: monofumarate
- HF: hemifumarate
- Mp: melting point

### Definitions

The term "tenofovir alafenamide" as used herein refers to 9-[(*R*)-2-[[(*S*)-[[(*S*)-1-(isopropoxycarbonyl)ethyl]amino]phenoxyphosphinyl]methoxy]propyl]adenine according to formula (I) disclosed herein above. A process for the preparation is disclosed in WO 02/008241 A2. Tenofovir alafenamide (INN/USAN, formerly GS-7340) is a nucleotide reverse transcriptase inhibitor and a prodrug of tenofovir. Developed by Gilead Sciences for use in the treatment of HIV infection and chronic hepatitis B, it is applied in the form of tenofovir alafenamide fumarate (TAF).

The term "tenofovir alafenamide hemifumarate" as used herein refers to the hemifumarate form of tenofovir alafenamide, having a chemical structure, wherein about two molecules of tenofovir alafenamide are associated with one molecule of fumaric acid. In one embodiment, the tenofovir alafenamide hemifumarate can be a crystalline salt, wherein protons have been transferred from at least some of the fumaric acid molecules to tenofovir alafenamide molecules. Since fumaric acid is a dicarboxylic acid, either one proton (i.e. hydrogen fumarate) or two protons (i.e. fumarate) can be transferred, dependent on the environment. In another embodiment, the tenofovir alafenamide hemifumarate can be a cocrystal, wherein substantially no protons have been transferred from fumaric acid molecules to tenofovir alafenamide molecules. Preferably, the term "tenofovir alafenamide hemifumarate" as used herein refers to a cocrystal.

The transfer of protons from one molecule to another in a crystal is dependent on the environment. Crystalline salts and cocrystals may be thought of as two ends of a proton transfer spectrum, where the salt has completed the proton transfer at one end and an absence of proton transfer exists for cocrystals at the other end.

The term "crystalline tenofovir alafenamide hemifumarate" as used herein refers to the crystalline form of tenofovir alafenamide hemifumarate disclosed in WO 2013/025788 A1. This form can be characterized by having a powder X-ray diffractogram comprising reflections at 2-Theta angles of (6.9 ± 0.2)°, (8.6 ± 0.2)°, (10.0 ± 0.2)°, (11.0 ± 0.2)°, (12.2 ± 0.2)°, (15.9 ± 0.2)°, (16.3 ± 0.2)°, (20.2 ± 0.2)° and (20.8 ± 0.2)°, when measured at a temperature in the range of from 20 to 30 °C with Cu-Kalpha_{1,2} radiation having a wavelength of 0.15419 nm.

As used herein, the term "measured at a temperature in the range of from 20 to 30 °C" refers to a measurement under standard conditions. Typically, standard conditions mean a temperature in the range of from 20 to 30 °C, i.e. at room temperature. Standard conditions can mean a temperature of about 22 °C. Standard conditions can also mean a temperature of about 25 °C. Typically, standard conditions can additionally mean a measurement under 20-80% relative humidity, preferably 30-70% relative humidity, more preferably 40-60% relative humidity and most preferably 50% relative humidity.

As used herein the term "room temperature" refers to a temperature in the range of from 20 to 30 °C.

The term "tenofovir alafenamide monofumarate" as used herein refers to the monofumarate form of 9-[(*R*)-2-[[(*S*)-[[(*S*)-1-(isopropoxycarbonyl)ethyl]amino]phenoxyphosphinyl] methoxy]propyl]adenine according to formula (II) disclosed herein having a chemical structure comprising about one molecule of tenofovir alafenamide per molecule fumaric acid. In one embodiment, the tenofovir alafenamide monofumarate can be a crystalline salt, wherein protons have been transferred from at least some of the fumaric acid molecules to tenofovir alafenamide molecules. Since fumaric acid is a dicarboxylic acid, either one proton (i.e. hydrogen fumarate) or two protons (i.e. fumarate) can be transferred, dependent on the environment. In another embodiment, the tenofovir alafenamide monofumarate can be a cocrystal, wherein substantially no protons have been transferred from fumaric acid molecules to tenofovir alafenamide molecules. Preferably, the term "tenofovir alafenamide monofumarate" as used herein refers to a cocrystal. Tenofovir alafenamide monofumarate has a molar ratio of tenofovir alafenamide and fumaric acid typically and preferably in a range of from about 1.0: 0.7 to 1.3, more preferably in a range of from about 1.0: 0.8 to 1.2, most preferably in a range of from about 1.0: 0.9 to 1.1 and in particular the molar ratio is about 1.0: 1.0.

The term "form I" as used herein when talking about a solid form of tenofovir alafenamide monofumarate refers to the solid form of tenofovir alafenamide monofumarate as obtained from example 4 in WO 02/08241 A2. This form can be characterized by having a PXRD comprising reflections at 2-Theta angles of (5.3 ± 0.1)°, (9.8 ± 0.1)°, (10.4 ± 0.1)°, (15.9 ± 0.1)°, (16.2 ± 0.1)° and (16.6 ± 0.1)°, when measured at a temperature in the range of from 20 to 30 °C with Cu-Kalpha_{1,2} radiation having a wavelength of 0.15419 nm. Figure 16 illustrates a representative PXRD of form I of tenofovir alafenamide monofumarate of WO 02/08241 A2 prepared according to reference example 1 herein.

The term "reflection" with regards to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 100 to 1000 atoms, whereas short-range order is over a few atoms only (see *"*Fundamentals of Powder Diffraction and Structural Characterization of Materials" by Vitalij K. Pecharsky and Peter Y. Zavalij, Kluwer Academic Publishers, 2003, page 3).

The term "essentially the same" with reference to powder X-ray diffraction means that variabilities in reflection positions and relative intensities of the reflections are to be taken into account. For example, a typical precision of the 2-Theta values is in the range of ± 0.2° 2-Theta, preferably in the range of ± 0.1° 2-theta. Thus, a reflection that usually appears at 7.3° 2-Theta for example can appear between 7.1° and 7.5° 2-theta, preferably between 7.2 and 7.4° 2-Theta on most X-ray diffractometers under standard conditions. Furthermore, one skilled in the art will appreciate that relative reflection intensities will show inter-apparatus variability as well as variability due to degree of crystallinity, preferred orientation, sample preparation and other factors known to those skilled in the art and should be taken as qualitative measure only.

As used herein, the term "substantially pure" with reference to a particular physical form means that the physical form includes at most 20%, preferably at most 10%, more preferably at most 5%, even more preferably at most 3% and most preferably at most 1% by weight of any other physical form of the compound.

The terms "physical form" and "solid form" are used interchangeably herein and refer to any crystalline and/or amorphous phase of a compound.

A crystalline form of tenofovir alafenamide monofumarate may be referred to herein as being characterized by graphical data "as shown in" a figure. Such data include, for example, PXRDs, Raman spectra, DSCs, TGAs and GMS isotherms. The person skilled in the art understands that factors such as variations in instrument type, response and variations in sample directionality, sample concentration, sample purity, sample history and sample preparation may lead to variations for such data when presented in graphical form, for example variations relating to the exact peak positions and intensities. However, a comparison of the graphical data in the figures herein with the graphical data generated for an unknown physical form and the confirmation that two sets of graphical data relate to the same crystal form is well within the knowledge of a person skilled in the art.

As used herein, the term "mother liquor" refers to the solution remaining after crystallization of a solid.

The term "non-hygroscopic" as used herein refers to a compound which shows a water uptake of at most 0.5 weight%, based on the weight of the compound, when measured with gravimetric moisture sorption at a relative humidity in the range of from 0 to 95% and a temperature of 25.0 ± 0.1 °C.

The term "anhydrous" as used herein, refers to a solid, where no water is coordinated in or accommodated by the crystal structure. However, an anhydrate may still comprise residual water due to surface adsorption, solvent inclusions and/or absorption in disordered regions.

The term "solvate" as used herein, refers to a solid, where one or more organic solvent(s) is/are coordinated in or accommodated by the crystal structure.

The term "isostructural solvate" as used herein, refers to solvates having the same space group with only small distortions of the unit cell dimensions and the same type of molecular network of the host molecule. Isostructural solvates as defined herein, only differ in the type of organic solvent present as guest molecule.

The term "desolvating" as used herein, means the at least partial removal of organic solvent from the crystal structure of the host molecule.

"Reduced pressure" as used herein means a pressure in the range of from 10 mbar to 900 mbar.

As used herein, the term "about" means within a statistically meaningful range of a value. Such a range can be within an order of magnitude, typically within 10%, more typically within 5%, even more typically within 1% and most typically within 0.1% of the indicated value or range. Sometimes, such a range can lie within the experimental error, typical of standard methods used for the measurement and/or determination of a given value or range.

### BRIEF DESCRIPTION OF THE FIGURES

**Figure 1****:** illustrates a representative PXRD of crystalline form II of tenofovir alafenamide monofumarate of the present invention. The x-axis shows the scattering angle in °2-theta, the y-axis shows the intensity of the scattered X-ray beam in counts of detected photons.
**Figure 2****:** illustrates a comparison of representative PXRDs of crystalline form II of tenofovir alafenamide monofumarate of the present invention (bottom) and form I of tenofovir alafenamide monofumarate of WO 02/08241 A2 (top). The x-axis shows the scattering angle in °2-theta. The intensities of the two PXRDs were first adapted so that the most intensive reflections of the two forms had similar intensities and then the PXRD of form I was shifted along the y-axis to separate the PXRDs for clarity reason. The y-axis is therefore arbitrary and was not labeled.
**Figure 3****:** illustrates a representative DSC curve of crystalline form II of tenofovir alafenamide monofumarate of the present invention. The x-axis shows the temperature in degree Celsius (°C), the y-axis shows the heat flow rate in Watt per gram (W/g) with endothermic peaks going up.
**Figure 4****:** illustrates a representative TGA curve of crystalline form II of tenofovir alafenamide monofumarate of the present invention. The x-axis shows the temperature in degree Celsius (°C), the y-axis shows the mass (loss) of the sample in percent (%).

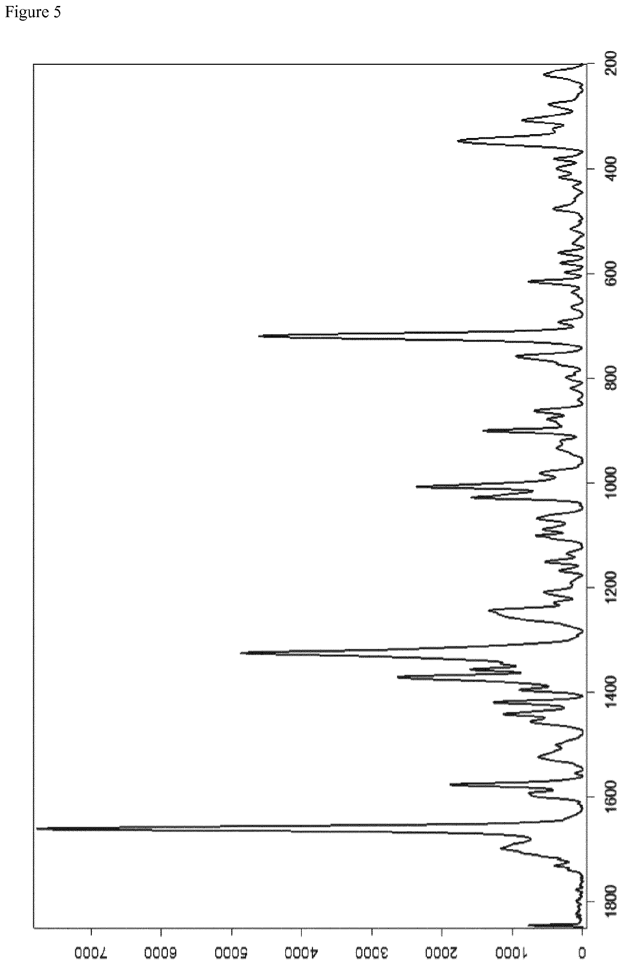

**Figure 5****:** illustrates a representative Raman spectrum of the crystalline form II of tenofovir alafenamide monofumarate of the present invention. The x-axis shows the wavenumbers in cm⁻¹, the y-axis shows the Raman intensity in counts.
**Figure 6****:** illustrates a representative PXRD of crystalline form III of tenofovir alafenamide monofumarate of the present invention. The x-axis shows the scattering angle in °2-theta, the y-axis shows the intensity of the scattered X-ray beam in counts of detected photons.
**Figure 7****:** illustrates a comparison of representative PXRDs of crystalline form III of tenofovir alafenamide monofumarate of the present invention (bottom) and form I of tenofovir alafenamide monofumarate of WO 02/08241 A2 (top). The x-axis shows the scattering angle in °2-theta. The intensities of the two PXRDs were first adapted so that the most intensive reflections of the two forms had similar intensities and then the PXRD of form I was shifted along the y-axis to separate the PXRDs for clarity reason. The y-axis is therefore arbitrary and was not labeled.
**Figure 8****:** illustrates a representative TGA curve of crystalline form III of tenofovir alafenamide monofumarate of the present invention. The x-axis shows the temperature in degree Celsius (°C), the y-axis shows the mass (loss) of the sample in percent (%).

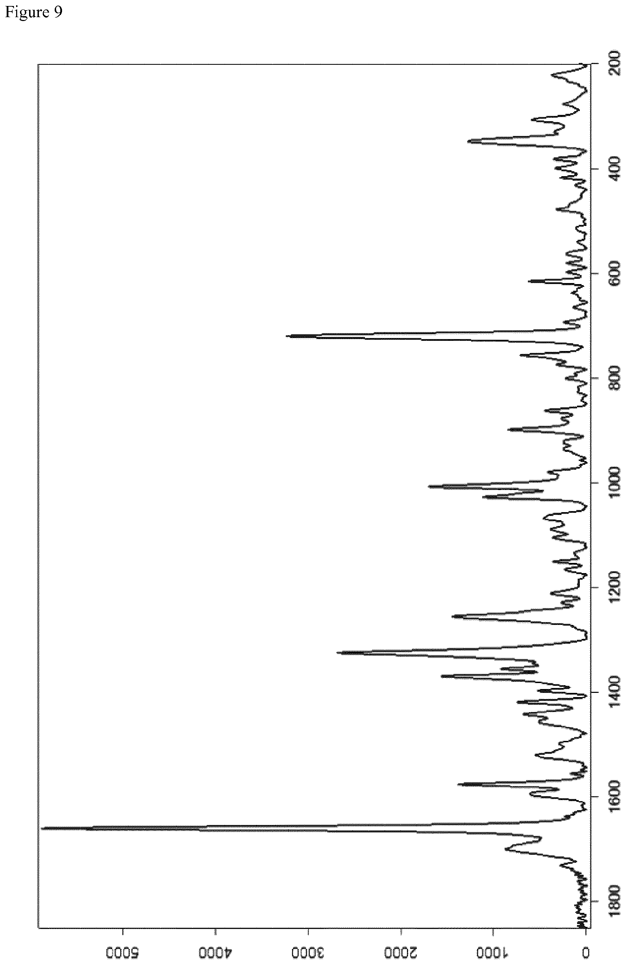

**Figure 9****:** illustrates a representative Raman spectrum of crystalline form III of tenofovir alafenamide monofumarate of the present invention. The x-axis shows the wavenumbers in cm⁻¹, the y-axis shows the Raman intensity in counts.

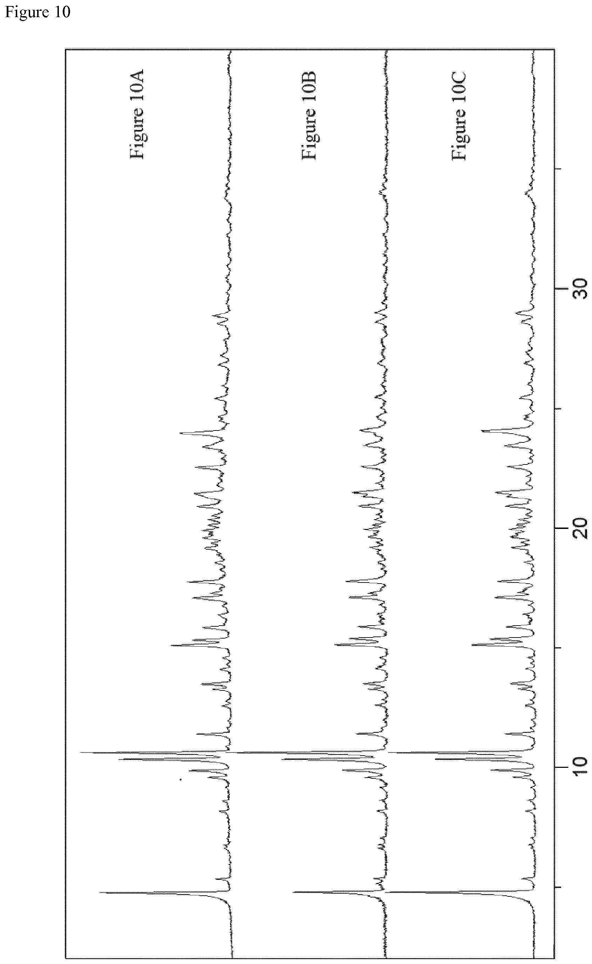

**Figure 10****:** illustrates a comparison of representative PXRDs of crystalline form S of tenofovir alafenamide monofumarate of the present invention prepared from n-butanol (top, Figure 10A), n-propanol (middle, Figure 10B) and isobutanol (bottom, Figure 10C). The x-axis shows the scattering angle in °2-theta. The intensities of the PXRDs were first adapted so that the most intensive reflections of the forms had similar intensities and then the PXRDs of form S prepared from *n*-butanol and n-propanol were shifted along the y-axis to separate the PXRDs for clarity reasons. The y-axis is therefore arbitrary and was not labeled.

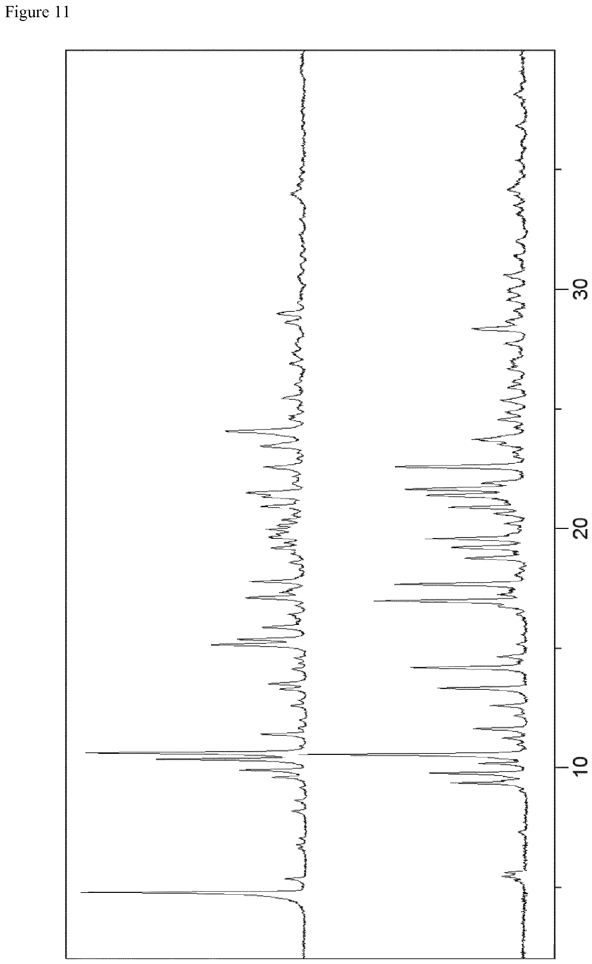

**Figure 11****:** illustrates a comparison of representative PXRDs of crystalline form S of tenofovir alafenamide monofumarate of the present invention prepared from isobutanol (top) and form III of tenofovir alafenamide monofumarate of the present invention (bottom). The x-axis shows the scattering angle in °2-theta. The intensities of the PXRDs were first adapted so that the most intensive reflections of the forms had similar intensities and then the PXRD of form S prepared from isobutanol was shifted along the y-axis to separate the PXRDs for clarity reasons. The y-axis is therefore arbitrary and was not labeled.

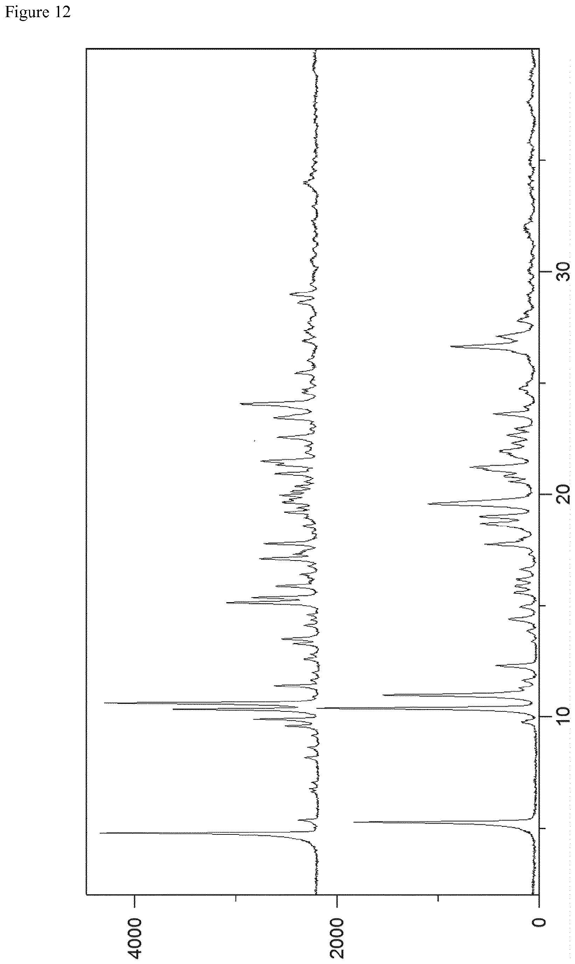

**Figure 12****:** illustrates a comparison of representative PXRDs of crystalline form S of tenofovir alafenamide monofumarate of the present invention prepared from isobutanol (top) and form I of tenofovir alafenamide monofumarate of WO 02/08241 A2 (bottom). The x-axis shows the scattering angle in °2-theta. The intensities of the PXRDs were first adapted so that the most intensive reflections of the forms had similar intensities and then the PXRD of form S prepared from isobutanol was shifted along the y-axis to separate the PXRDs for clarity reasons. The y-axis is therefore arbitrary and was not labeled.
**Figure 13****:** illustrates a representative DSC curve of crystalline form S of tenofovir alafenamide monofumarate of the present invention prepared from isobutanol. The x-axis shows the temperature in degree Celsius (°C), the y-axis shows the heat flow rate in Watt per gram (W/g) with endothermic peaks going up.
**Figure 14****:** illustrates a representative TGA curve of crystalline form S of tenofovir alafenamide monofumarate of the present invention prepared from isobutanol. The x-axis shows the temperature in degree Celsius (°C), the y-axis shows the mass (loss) of the sample in percent (%).

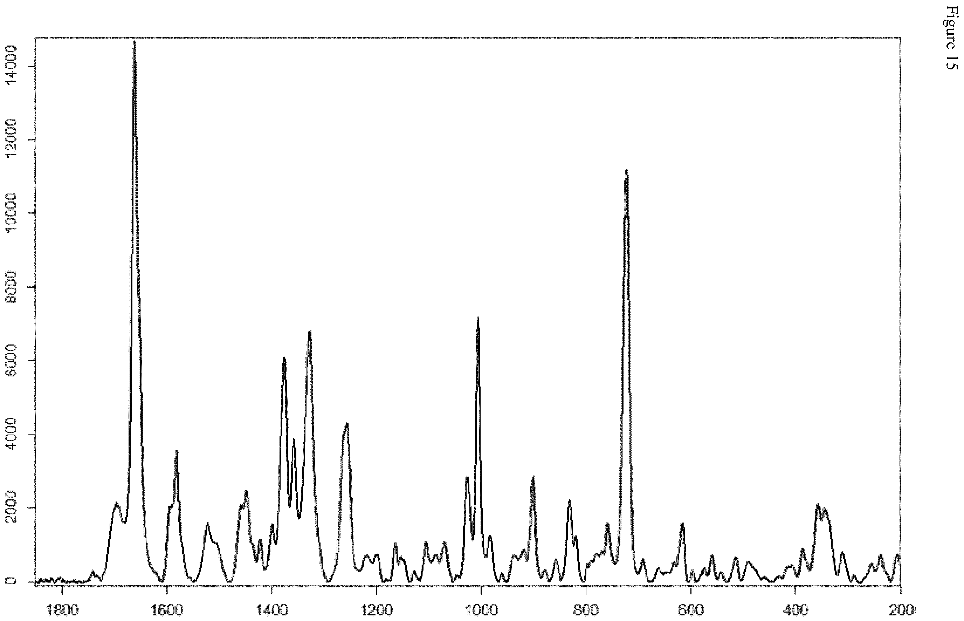

**Figure 15****:** illustrates a representative Raman spectrum of the crystalline form S of tenofovir alafenamide monofumarate of the present invention prepared from isobutanol. The x-axis shows the wavenumbers in cm⁻¹, the y-axis shows the Raman intensity in counts.

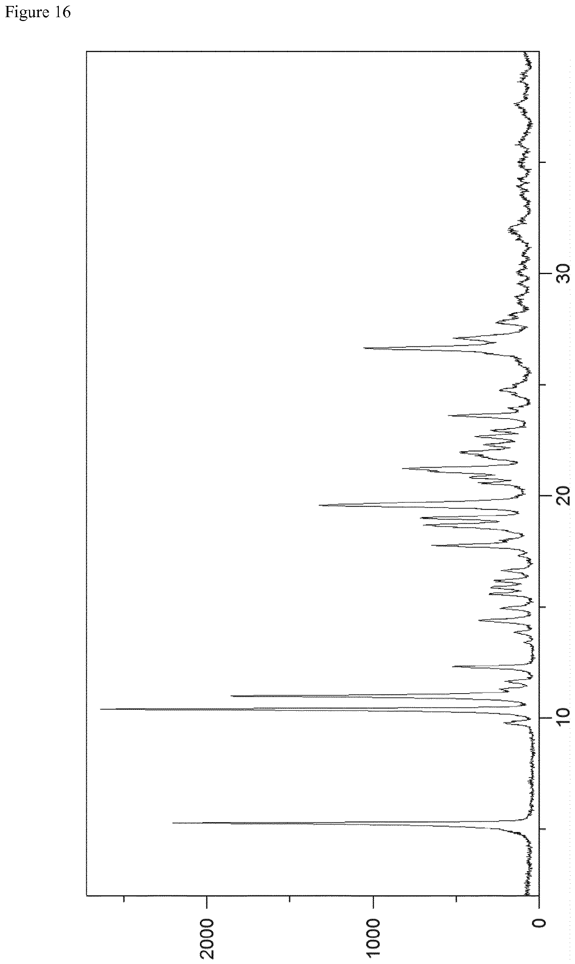

**Figure 16****:** illustrates a representative PXRD of form I of tenofovir alafenamide monofumarate of WO 02/08241 A2 prepared according to reference example 1 herein. The x-axis shows the scattering angle in °2-theta, the y-axis shows the intensity of the scattered X-ray beam in counts of detected photons.
**Figure 17****:** illustrates a representative DSC curve of form I of tenofovir alafenamide monofumarate of WO 02/08241 A2 prepared according to reference example 1 herein. The x-axis shows the temperature in degree Celsius (°C), the y-axis shows the heat flow rate in Watt per gram (W/g) with endothermic peaks going up.
**Figure 18****:** illustrates a representative TGA curve of form I of WO 02/08241 A2 prepared according to reference example 1 herein. The x-axis shows the temperature in degree Celsius (°C), the y-axis shows the mass (loss) of the sample in percent (%).

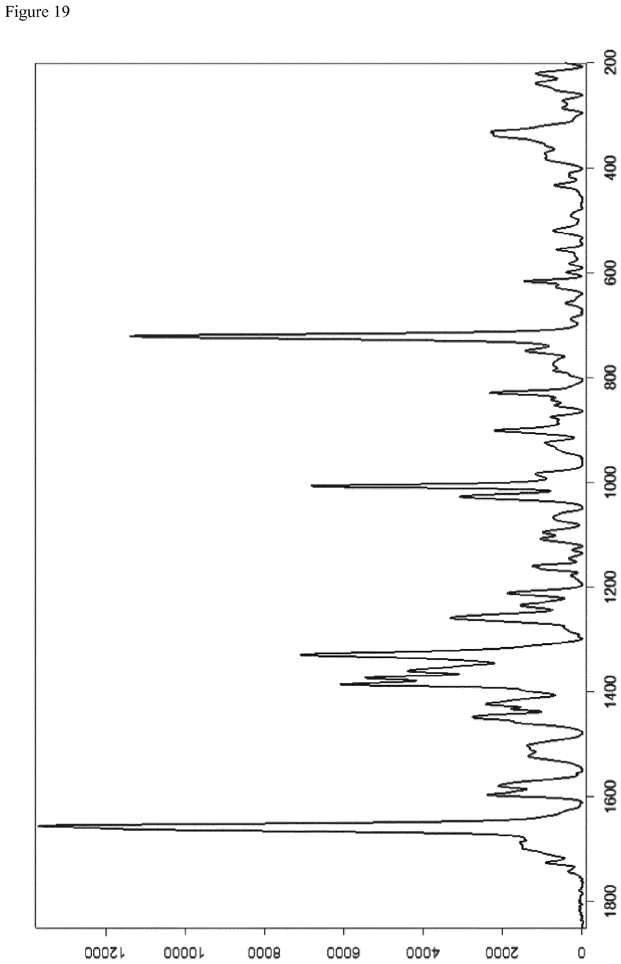

**Figure 19****:** illustrates a representative Raman spectrum of crystalline form I of tenofovir alafenamide monofumarate of WO 02/08241 A2 prepared according to reference example 1 herein. The x-axis shows the wavenumbers in cm⁻¹, the y-axis shows the Raman intensity in counts.
**Figure 20A****:** illustrates comparisons of representative GMS isotherms of tenofovir alafenamide monofumarate form I of WO 02/08241 A2 (squares), form II of the present invention (circles) and form III of the present invention (triangles) during the sorption cycle from 0 to 95% relative humidity. The x-axis displays the relative humidity in percent (%) measured at a temperature of (25.0 ± 0.1) °C, the y-axis displays the equilibrium mass change in percent (%). The values are displayed as uncorrected values.

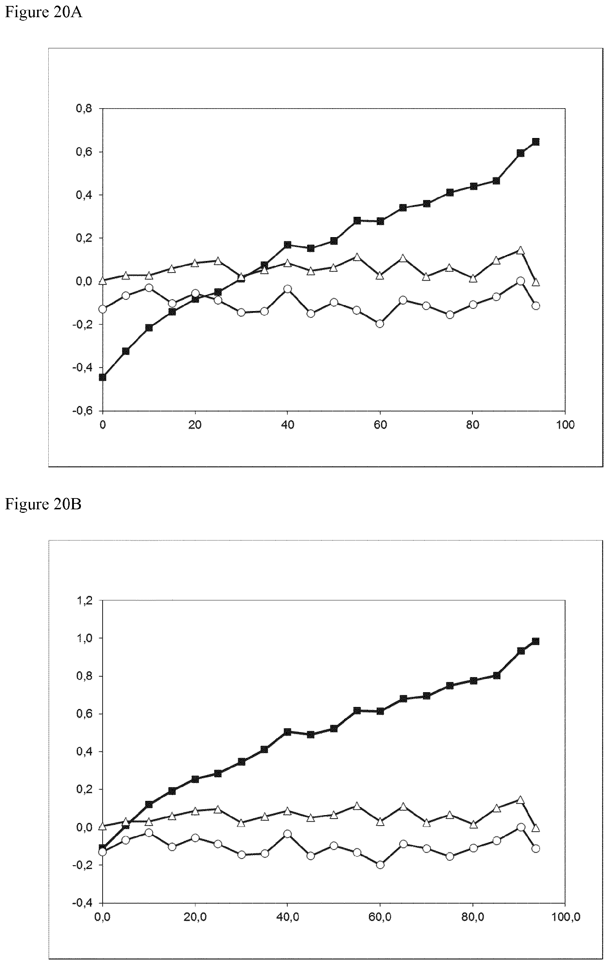

**Figure 20B****:** illustrates comparisons of representative GMS isotherms of tenofovir alafenamide monofumarate form I of WO 02/08241 A2 (squares), form II of the present invention (circles) and form III of the present invention (triangles) during the sorption cycle from 0 to 95% relative humidity. The x-axis displays the relative humidity in percent (%) measured at a temperature of (25.0 ± 0.1) °C, the y-axis displays the equilibrium mass changes in percent (%). The values are corrected to water contents determined at the end of the experiment (Form I: 0.50 weight% H₂O, Form II: 0.08 weight% H₂O, Form III: 0.16 weight% H₂O).

### DETAILED DESCRIPTION OF THE INVENTION

The present invention provides crystalline forms of tenofovir alafenamide monofumarate, herein also designated "form II", "form III" and "form S".

Tenofovir alafenamide monofumarate can be represented by the following chemical structure according to formula (II):

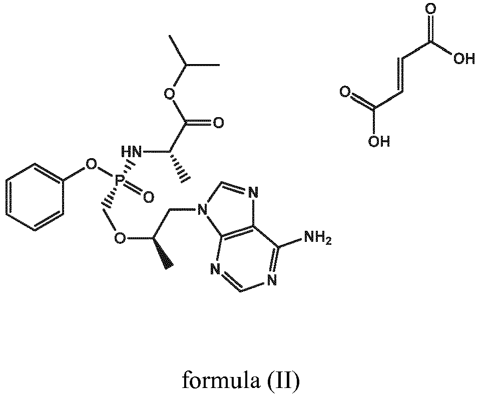

Tenofovir alafenamide monofumarate has a molar ratio of tenofovir alafenamide and fumaric acid typically and preferably in a range of from about 1.0: 0.7 to 1.3, more preferably in a range of from about 1.0: 0.8 to 1.2, and in particular the molar ratio is about 1.0: 1.0.

A process for the preparation of tenofovir alafenamide monofumarate is described in example 4 of WO 02/08241 A2. The process comprises charging a reactor with tenofovir alafenamide, fumaric acid and acetonitrile, dissolving the solids by heating to reflux temperature, hot filtration of the obtained solution and cooling to a temperature of 5 °C for 16 hours. The product is isolated by filtration, rinsed with acetonitrile and dried. According to example 4, a white powder having a melting point in the range of from 119.7 to 121.1 °C was obtained.

Additional processes for the preparation of tenofovir alafenamide monofumarate are disclosed in WO 2015/107451 A2. The last step of example 19 for example comprises a reaction of tenofovir alafenamide with fumaric acid in ethanol. The mixture is first heated to 65 °C and then in a subsequent step cooled to 5 °C. In example 20 of the same application tenofovir alafenamide is reacted with fumaric acid in the presence of methanol. The reaction mass was heated to reflux before it was slowly cooled to room temperature and further cooled to 0°C. However, the application is silent about the solid forms obtained. Repetition of these examples revealed that in both cases the same crystalline form of tenofovir alafenamide monofumarate, namely the known form from WO 02/08241 A2 herein also designated form I is obtained (see reference examples 3 and 4 hereinafter).

Example 1 of CN 105237571 A discloses a process for the preparation of tenofovir alafenamide monofumarate comprising reacting tenofovir alafenamide with fumaric acid in the presence of acetonitrile. The reaction mixture was heated to reflux, cooled to 22 °C and stirred at this temperature overnight. Again, the application is silent about the solid form obtained. Repetition of this example yielded a mixture consisting of the known form of tenofovir alafenamide monofumarate of WO 02/08241 A2, which is herein also designated form I and tenofovir alafenamide hemifumarate of WO 2013/025788 A1 (see reference example 4).

According to WO 2013/025788 A1, the crystalline hemifumarate form can be prepared by subjecting a solution comprising a suitable solvent, fumaric acid tenofovir alafenamide and optionally one or more seeds of tenofovir alafenamide hemifumarate to conditions that provide for the crystallization of fumaric acid and tenofovir alafenamide. A specific example for the preparation of tenofovir alafenamide hemifumarate is e.g. provided in example 3 of the application, where a solution comprising tenofovir alafenamide (10 g), fumaric acid (1.22 g) and acetonitrile (100 mL) was heated to 70-75 °C to dissolve the solids. Any undissolved particulates were removed by filtration through a cartridge filter. The solution was cooled to 60-65 °C and seeded with 1% (by weight) of tenofovir alafenamide hemifumarate. The slurry was aged for 30 minutes and cooled to 0-5 °C over 2 hours. The temperature was maintained for 1-18 hours and the resulting slurry was filtered and washed with 2 mL of cold acetonitrile (0-5 °C). The solids were dried under vacuum at 50 °C to provide the hemifumarate form of tenofovir alafenamide.

WO 2013/025788 A1 further describes in paragraph [0071] that the tenofovir alafenamide monofumarate form of WO 02/08241 A2, herein also designated "form I", at least partially in some solvents even completely transforms to the therein disclosed tenofovir alafenamide hemifumarate form when slurried in organic solvents.

The inventors of the present invention now surprisingly found that slurrying form I of tenofovir alafenamide monofumarate in acetonitrile for an unusual long time (i.e. for several days), leads to a different crystalline form of tenofovir alafenamide monofumarate instead, namely form II. Both, form I of WO 02/08241 A2 and form II of the present invention are anhydrous and non-solvated forms of tenofovir alafenamide monofumarate and therefore can be considered as polymorphs in the strict sense.

Polymorph II of the present invention possesses the higher melting point and the higher heat of fusion than polymorph I of WO 02/08241 A2. Therefore, according to Burger and Ramberger's heat of fusion rule, form I and form II are monotropic with form II being the thermodynamically stable form below its melting point (A. Burger, R. Ramberger, "On the Polymorphism of Pharmaceuticals and Other Molecular Crystals. I; Mikrochimica Acta [Wien] 1979 II, 259-271). The fact that the monofumarate form I transforms into the monofumarate form II of tenofovir alafenamide when slurried in a solvent like acetonitrile for sufficient long time also indicates that form II is the thermodynamically stable form of tenofovir alafenamide monofumarate.

The usage of the thermodynamically most stable form of a compound is highly appreciated as polymorphic conversions, which may occur during manufacturing process and storage of a drug substance can be excluded, when the stable form is used. This ensures reliable bioavailability and therefore consistent efficacy of a drug product. The inventors of the present invention also provided a crystalline solvate of tenofovir alafenamide monofumarate, herein designated "form S". Form S can be obtained by crystallization from solvents selected from alcohols such as *n-*propanol, n-butanol and isobutanol. Depending on the solvent used, form S may be present as n-propanol, n-butanol or isobutanol solvate, all of them being isostructural solvates resulting in substantially the same PXRD pattern. Depending on the desolvation conditions either form I of WO 02/08241 A2 or form III of the present invention may be obtained from form S. For example, desolvation upon moisture contact leads to form I, whereas desolvation at elevated temperature leads to form III. In particular, the inventors of the present invention found that storing form S of tenofovir alafenamide monofumarate in vacuum (20-30 mbar) at a temperature of about 110 °C for about 6 hours leads to a further crystalline form of tenofovir alafenamide monofumarate, namely form III.

Hence, having form S in hand, on the one hand provides a selective process for the preparation of form I and on the other hand for the first time allows the production of form III of tenofovir alafenamide monofumarate. Hence, form S of the present invention is a valuable intermediate for the preparation of crystalline forms of tenofovir alafenamide monofumarate, in particular form III. A further advantageous property of crystalline form S is, that it is physically stable against pressure.

In addition, the inventors of the present invention surprisingly found that in contrast to form I of WO 02/08241 A2, both form II and form III of tenofovir alafenamide monofumarate of the present invention practically show no interaction with water vapor at all.

Due to their non-hygroscopic behavior, form II and form III of the present invention are preferred over form I of WO 02/08241 A2 as the physicochemical properties of forms II and III are preserved regardless the relative humidity of the surrounding atmosphere, which facilitates easier and more reliable manufacturing processes as well as easier storage of a pharmaceutical product containing these forms.

Crystalline forms II, III and S of tenofovir alafenamide monofumarate of the present invention may be characterized by analytical methods well known in the field of the pharmaceutical industry for characterizing solids. Such methods comprise but are not limited to PXRD, Raman, DSC, TGA and GMS. The crystalline forms of the present invention may be characterized by one of the aforementioned analytical methods or by combining two or more of them. In particular, they may be characterized by any one of the following embodiments or by combining two or more of the following embodiments.

Hence, in a first aspect the present invention relates to a crystalline form of tenofovir alafenamide monofumarate (form II) characterized by having a PXRD comprising reflections at 2-Theta angles of:
(7.3 ± 0.2)°, (9.4 ± 0.1)° and (10.1 ± 0.1)°; or
(7.3 ± 0.2)°, (9.4 ± 0.1)°, (10.1 ± 0.1)° and (13.0 ± 0.1)°; or
(5.6 ± 0.1)°, (7.3 ± 0.2)°, (9.4 ± 0.1)°, (10.1 ± 0.1)° and (13.0 ± 0.1)°; or
(5.6 ± 0.1)°, (7.3 ± 0.2)°, (9.4 ± 0.1)°, (10.1 ± 0.1)°, (13.0 ± 0.1)° and (28.4 ± 0.1)°;
when measured at a temperature in the range of from 20 to 30 °C with Cu-Kalpha_{1,2} radiation having a wavelength of 0.15419 nm.

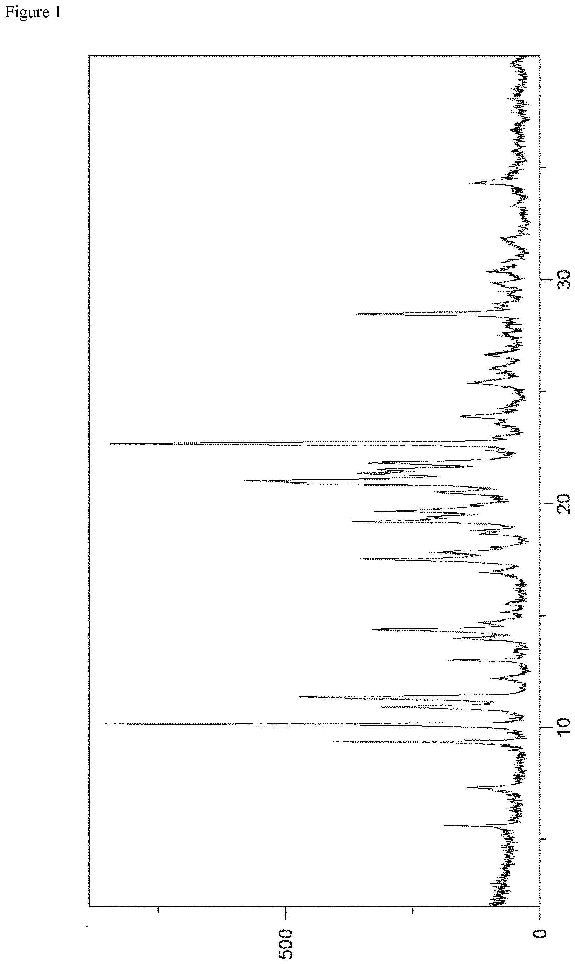

In another embodiment, the present invention relates to a crystalline form of tenofovir alafenamide monofumarate (form II) characterized by having a PXRD essentially the same as shown in figure 1 of the present invention, when measured at a temperature in the range of from 20 to 30 °C with Cu-Kalpha_{1,2} radiation having a wavelength of 0.15419 nm.

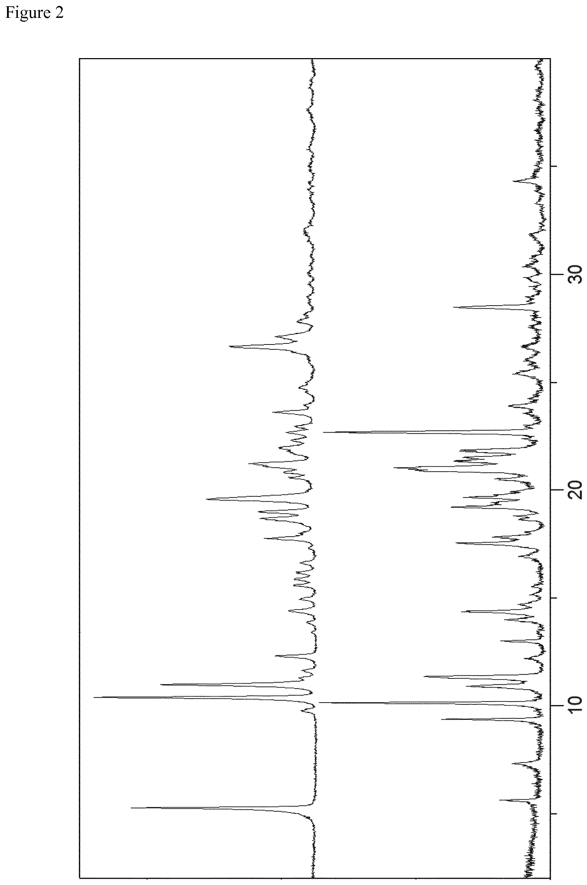

The PXRD of form II of the present invention can be clearly distinguished from the PXRD of form I of WO 02/08241 A2 (see also PXRD overlay displayed in figure 2 herein). For instance, Form II shows a peak at (7.3 ± 0.2)° 2-Theta, whereas form I shows no reflection in the same range. On the other hand, form II shows no reflection at (5.3 ± 0.1)° 2-Theta, whereas form I shows an intensive reflection in this range.

In a further preferred embodiment, the present invention relates to a crystalline form of tenofovir alafenamide monofumarate (form II) characterized by showing an endotherm with an onset temperature in the range of from 122.0 to 122.5 °C, preferably of about 122 °C, more preferably of 122.3°, when measured with DSC at a heating rate of 10 K/min.

More preferably, the present invention relates to a crystalline form of tenofovir alafenamide monofumarate (form II) characterized by having a heat of fusion of at least 102.0 J/g, preferably of at least 103.0 J/g, even more preferably of at least 104.0 J/g, such as of at least 104.5 J/g, when measured with DSC at a heating rate of 10 K/min. Most preferably, the crystalline tenofovir alafenamide monofumarate form II is characterized by a heat of fusion of 104.8 J/g, when measured with DSC at a heating rate of 10 K/min.

In another embodiment, the present invention relates to a crystalline form of tenofovir alafenamide monofumarate (form II) characterized by showing a mass loss of not more than 2.0 weight%, preferably not more than 1.5 weight%, more preferably not more than 1.0 weight% and most preferably not more than 0.5 weight%, such as not more than 0.3 weight%, based on the weight of the crystalline form, when measured with TGA at a temperature in the range of from 25 to 130 °C and a heating rate of 10 K/min.

In yet another embodiment, the present invention relates to a crystalline form of tenofovir alafenamide monofumarate (form II) characterized by showing a mass change of not more than 2.0 weight%, preferably of not more than 1.5 weight%, more preferably of not more than 1.0 weight% and most preferably of not more than 0.5 weight%, for example of not more than 0.4 weight%, 0.3 weight%, 0.2 weight% or 0.1 weight%, based on the weight of the crystalline form, when measured with GMS at a relative humidity in the range of from 0 to 95% and a temperature of (25.0 ± 0.1)°C. Preferably, form II of the present invention is anhydrous, more preferably it is non-hygroscopic.

In a further embodiment, the present invention relates to a crystalline form of tenofovir alafenamide monofumarate (form II) characterized by having a Raman spectrum comprising peaks at wavenumbers of:
(347 ± 2) cm⁻¹, (477 ± 2) cm⁻¹, (758 ± 2) cm⁻¹, (863 ± 2) cm⁻¹ and (1244 ± 2) cm⁻¹; or
(347 ± 2) cm⁻¹, (400 ± 2) cm⁻¹, (477 ± 2) cm⁻¹, (561 ± 2) cm⁻¹, (758 ± 2) cm⁻¹, (863 ± 2) cm⁻¹, (878 ± 2) cm⁻¹ and (1244 ± 2) cm⁻¹; or
(347 ± 2) cm⁻¹, (400 ± 2) cm⁻¹, (477 ± 2) cm⁻¹, (561 ± 2) cm⁻¹, (758 ± 2) cm⁻¹, (863 ± 2) cm⁻¹, (878 ± 2) cm⁻¹, (1089 ± 2) cm⁻¹, (1151 ± 2) cm⁻¹, (1244 ± 2) cm⁻¹, (1395 ± 2) cm⁻¹, (1419 ± 2) cm⁻¹ and (1442 ± 2) cm⁻¹;
when measured at a temperature in the range of from 20 to 30 °C and a wavelength of 785 nm.

The Raman spectrum of form II of the present invention can be clearly distinguished from the Raman spectrum of form I of WO 02/08241 A2. For example, form II shows characteristic peaks at (347 ± 2) cm⁻¹, (477 ± 2) cm⁻¹, (863 ± 2) cm⁻¹ and (1151 ± 2) cm⁻¹, whereas form I shows no peaks in the same ranges. On the other hand, form II shows no peaks at (239 ± 2) cm⁻¹, (829 ± 2) cm⁻¹, (1385 ± 2) cm⁻¹ and (1448 ± 2) cm⁻¹, whereas form I shows peaks in these ranges.

In one embodiment, a composition comprising form II of tenofovir alafenamide monofumarate of the present invention is essentially free of any other physical forms of tenofovir alafenamide monofumarate and/or hemifumarate. For example, a composition comprising form II of tenofovir alafenamide of the present invention comprises at most 20 weight%, preferably at most 10 weight%, more preferably at most 5 weight%, even more preferably at most 2 weight% and most preferably at most 1 weight% of any other physical form of tenofovir alafenamide monofumarate and/or hemifumarate, based on the weight of the composition. Preferably, the any other physical form of tenofovir alafenamide monofumarate is form I of WO 02/08241 A2 and the any other physical form of tenofovir alafenamide hemifumarate is the crystalline tenofovir alafenamide hemifumarate described in WO 2013/025788 A1.

In another aspect, the present invention relates to a process for the preparation of crystalline form II of tenofovir alafenamide monofumarate of the present invention or the composition comprising the same as defined herein comprising:
(i) providing tenofovir alafenamide monofumarate in solid form;
(ii) slurrying tenofovir alafenamide monofumarate provided in step (i) in a solvent comprising acetonitrile for at least 10 days;
(iii)optionally separating at least a part of the crystals obtained in step (ii) from their mother liquor;
(iv)optionally washing the isolated crystals obtained in step (iii);
(v) optionally drying the crystals obtained in any one of steps (ii) to (iv);

The solid tenofovir alafenamide monofumarate starting material can be prepared according to the procedure disclosed in WO 02/08241 A2 (compound V with development code GS-7340-02 corresponds to tenofovir alafenamide monofumarate). Following the procedure for tenofovir alafenamide monofumarate production described in example 4 of WO 02/08241 A2 leads to a solid form, which is herein also designated "form I" (see also reference example 1 herein).

In a preferred embodiment, tenofovir alafenamide monofumarate form I is used as starting material in step (i) of the above described process. In another preferred embodiment, tenofovir alafenamide monofumarate form III is used as starting material in step (i) of the above described process. In another preferred embodiment, tenofovir alafenamide monofumarate form S is used as starting material in step (i) of the above described process.

The solid starting material provided in step (i) is slurried in a solvent comprising acetonitrile. The solvent may comprise additional organic solvents and/or water. However, most preferably acetonitrile is the only solvent present in the slurry. The tenofovir alafenamide monofumarate concentration of the suspension is preferably in the range of from about 10 to 200 g/L, more preferably from about 50 to 150 g/L and most preferably from about 75 to 125 g/L, for example the concentration is about 100 g/L. Preferably, slurrying is performed at room temperature but depending on the applied concentration may also be conducted at elevated temperature for example at a temperature in the range of from about 40 to 80 °C, preferably from about 40 to 60 °C. Slurrying emcompasses any kind of movement of the solid material suspended in the solvent caused by, but not limited to e.g. agitation, stirring, mixing, shaking, vibration, sonication, wet milling and the like. Slurrying is conducted for at least 10 days, such as 10 days, 11 days or longer. The skilled person may monitor the conversion of the applied solid form of tenofovir alafenamide monofumarate to form II by withdrawing samples from the slurry and analyzing the sample by powder X-ray diffraction.

Once, tenofovir alafenamide monofumarate form II is obtained in essentially pure form, at least a part of the crystals are separated from their mother liquor. Preferably, the crystals are separated from their mother liquor by any conventional method such as filtration, centrifugation, solvent evaporation or decantation, more preferably by filtration or centrifugation and most preferably by filtration.

Optionally, in a further step the isolated crystals are washed with a suitable solvent, for example an organic solvent and/or water. Suitable organic solvents comprise but are not limited to acetonitrile.

The obtained crystals may optionally be dried. Drying may be performed at a temperature of about 100 °C or less, preferably of about 80 °C or less, more preferably of about 60 °C or less and most preferably of about 40 °C or less. Typically, drying is performed at about room temperature. Drying may be performed for a period in the range of from about 1 to 72 hours, preferably from about 2 to 48 hours, more preferably from about 4 to 24 hours and most preferably from about 6 to 18 hours. Drying may be performed at ambient pressure and/or under reduced pressure. Preferably, drying is performed at a pressure of about 100 mbar or less, more preferably of about 50 mbar or less and most preferably of about 30 mbar or less, for example a vacuum of about 20 mbar or less.

Tenofovir alafenamide monofumarate form II prepared according to the above described procedure may be used as seed crystals in a crystallization procedure. For example, tenofovir alafenamide monofumarate form I may be dissolved in a suitable solvent such as ethyl acetate upon heating and the obtained solution may be seeded with form II crystals in order to initiate crystallization of form II.

Therefore, in a further aspect the invention relates to the use of tenofovir alafenamide monofumarate form II of the present invention as seed crystals in a crystallization process for the preparation of tenofovir alafenamide monofumarate form II.

In another aspect, the present invention relates to a crystalline form of tenofovir alafenamide monofumarate (form III) characterized by having a PXRD comprising reflections at 2-Theta angles of:
(5.6 ± 0.1)°, (7.3 ± 0.2)° and (10.5 ± 0.1)°; or
(5.6 ± 0.1)°, (7.3 ± 0.2)°, (10.5 ± 0.1)° and (12.6 ± 0.1)°; or
(5.6 ± 0.1)°, (7.3 ± 0.2)°, (10.5 ± 0.1)°, (12.6 ± 0.1)° and (17.0 ± 0.1)°; or
(5.6 ± 0.1)°, (7.3 ± 0.2)°, (9.4 ± 0.1)°, (10.5 ± 0.1)°, (12.6 ± 0.1)° and (17.0 ± 0.1)°;
when measured at a temperature in the range of from 20 to 30 °C with Cu-Kalpha_{1,2} radiation having a wavelength of 0.15419 nm.

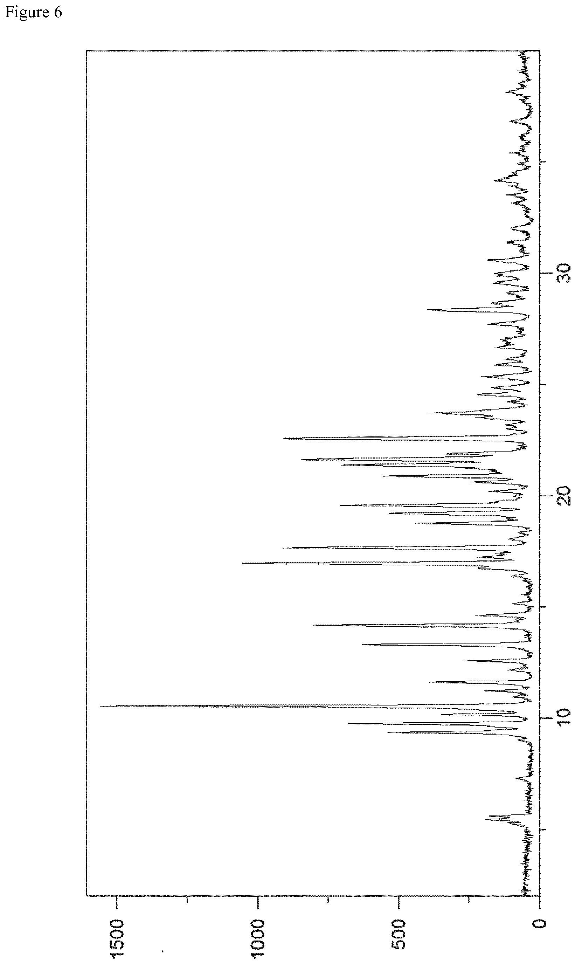

In one embodiment, the present invention relates to a crystalline form of tenofovir alafenamide monofumarate (form III) characterized by having a PXRD essentially the same as shown in figure 6 of the present invention, when measured at a temperature in the range of from 20 to 30 °C with Cu-Kalpha_{1,2} radiation having a wavelength of 0.15419 nm.

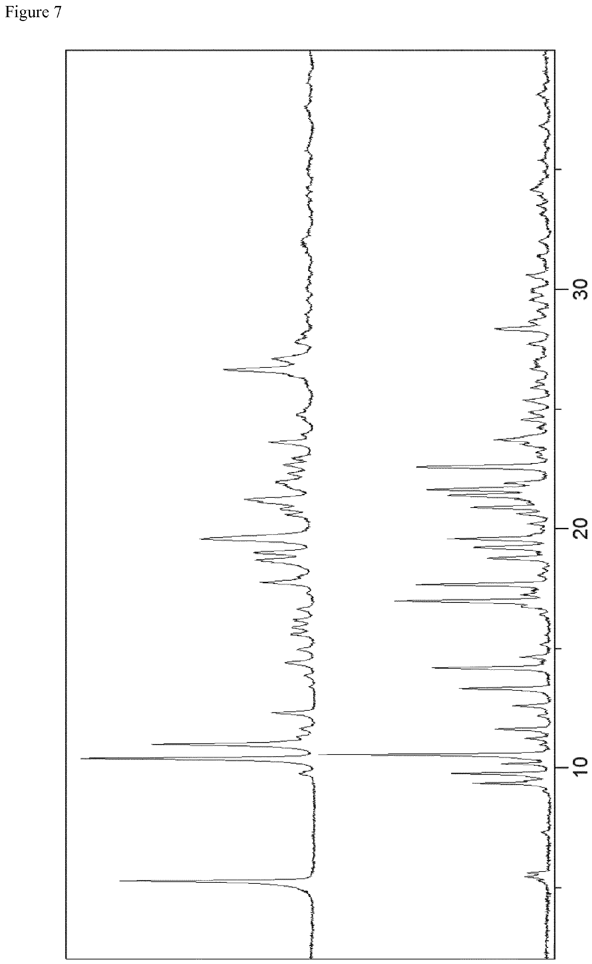

The PXRD of form III of the present invention can be clearly distinguished from the PXRD of form I of WO 02/08241 A2 (see also PXRD overlay displayed in figure 7 herein). For instance, Form III shows peaks at (5.6 ± 0.1)°,(7.3 ± 0.2)° and (9.4 ± 0.1)° 2-Theta, whereas form I shows no reflection in the same ranges.

In another embodiment, the present invention relates to a crystalline form of tenofovir alafenamide monofumarate (form III) characterized by showing a mass loss of not more than 2.0 weight%, preferably not more than 1.5 weight%, more preferably not more than 1.0 weight% and most preferably not more than 0.5 weight%, such as not more than 0.4 weight%, 0.3 weight%, 0.2 weight% or 0.1 weight%, based on the weight of the crystalline form, when measured with TGA at a temperature in the range of from 25 to 130 °C and a heating rate of 10 K/min.

In yet another embodiment, the present invention relates to a crystalline form of tenofovir alafenamide monofumarate (form III) characterized by showing a mass change of not more than 2.0 weight%, preferably of not more than 1.5 weight%, more preferably of not more than 1.0 weight% and most preferably of not more than 0.5 weight%, for example of not more than 0.4 weight%, 0.3 weight%, 0.2 weight% or 0.1 weight%, based on the weight of the crystalline form, when measured with GMS at a relative humidity in the range of from 0 to 95% and a temperature of (25.0 ± 0.1)°C. Preferably, form III of the present invention is anhydrous, more preferably it is non-hygroscopic.

In a further embodiment, the present invention relates to a crystalline form of tenofovir alafenamide monofumarate (form III) characterized by having a Raman spectrum comprising peaks at wavenumbers of:
(348 ± 2) cm⁻¹, (478 ± 2) cm⁻¹, (756 ± 2) cm⁻¹, (862 ± 2) cm⁻¹ and (1256 ± 2) cm⁻¹; or
(348 ± 2) cm⁻¹, (400 ± 2) cm⁻¹, (478 ± 2) cm⁻¹, (563 ± 2) cm⁻¹, (756 ± 2) cm⁻¹, (862 ± 2) cm⁻¹, (1089 ± 2) cm⁻¹ and (1256 ± 2) cm⁻¹; or
(348 ± 2) cm⁻¹, (400 ± 2) cm⁻¹, (478 ± 2) cm⁻¹, (563 ± 2) cm⁻¹, (756 ± 2) cm⁻¹, (801 ± 2) cm⁻¹, (862 ± 2) cm⁻¹, (1089 ± 2) cm⁻¹, (1151 ± 2) cm⁻¹, (1256 ± 2) cm⁻¹, (1397 ± 2) cm⁻¹, (1419 ± 2) cm⁻¹ and (1442 ± 2) cm⁻¹;
when measured at a temperature in the range of from 20 to 30 °C and a wavelength of 785 nm.

The Raman spectrum of form III of the present invention can be clearly distinguished from the Raman spectrum of form I of WO 02/08241 A2. For example, form III shows characteristic peaks at (348 ± 2) cm⁻¹, (478 ± 2) cm⁻¹, (862 ± 2) cm⁻¹ and (1151 ± 2) cm⁻¹, whereas form I shows no peaks in the same ranges. On the other hand, form III shows no peaks at (239 ± 2) cm⁻¹, (829 ± 2) cm⁻¹, (1385 ± 2) cm⁻¹ and (1448 ± 2) cm⁻¹, whereas form I shows peaks in these ranges.

In one embodiment, a composition comprising form III of tenofovir alafenamide monofumarate of the present invention is essentially free of any other physical forms of tenofovir alafenamide monofumarate and/or hemifumarate. For example, a composition comprising form III of tenofovir alafenamide of the present invention comprises at most 20 weight%, preferably at most 10 weight%, more preferably at most 5 weight%, even more preferably at most 2 weight% and most preferably at most 1 weight% of any other physical form of tenofovir alafenamide monofumarate and/or hemifumarate, based on the weight of the composition. Preferably, the any other physical form of tenofovir alafenamide monofumarate is form I of WO 02/08241 A2 and the any other physical form of tenofovir alafenamide hemifumarate is the crystalline tenofovir alafenamide hemifumarate described in WO 2013/025788 A1.

In another aspect, the present invention relates to a process for the preparation of crystalline form III of tenofovir alafenamide monofumarate of the present invention or the composition comprising the same as defined herein comprising:
(i) providing a crystalline solvate of tenofovir alafenamide monofumarate (form S) as defined herein;
(ii) at least partially desolvating the crystalline solvate provided in step (i) at a temperature in the range of from 100 to 115 °C;

The crystalline solvate of tenofovir alafenamide monofumarate (form S) provided in step (i) can be prepared according to the general procedure disclosed hereinafter for form S production. Particular examples for form S production are also described in examples 3 to 6 herein.

Form III is prepared by at least partially desolvating form S of tenofovir alafenamide monofumarate at elevated temperature. The desolvation is performed by subjecting form S to a temperature of at least 100 °C, preferably of at least 105 °C and most preferably of at least 110 °C, with the proviso that the temperature does not exceed 115 °C. In addition, desolvation may be performed at reduced pressure, for example by applying a vacuum of about 100 mbar or less, more preferably of about 50 mbar or less and most preferably of about 30 mbar or less, for example of about 20 mbar or less. Form S is subjected to the aforementioned desolvation conditions for a period in the range of from about 1 to 72 hours, preferably from about 2 to 48 hours, more preferably from about 4 to 24 hours and most preferably from about 4 to 12 hours. The skilled person may monitor the conversion of tenofovir alafenamide form S to form III by withdrawing samples and analyzing them by powder X-ray diffraction.

In yet another aspect, the present invention relates to a crystalline solvate of tenofovir alafenamide monofumarate (form S) characterized by having a PXRD comprising reflections at 2-Theta angles of:
(4.8 ± 0.2)°, (10.3 ± 0.2)° and (10.6 ± 0.2)°; or
(4.8 ± 0.2)°, (10.3 ± 0.2)°, (10.6 ± 0.2)° and (24.0 ± 0.2)°; or
(4.8 ± 0.2)°, (10.3 ± 0.2)°, (10.6 ± 0.2)°, (15.1 ± 0.2)° and (24.0 ± 0.2)°; or
(4.8 ± 0.2)°, (10.3 ± 0.2)°, (10.6 ± 0.2)°, (15.1 ± 0.2)°, (15.3 ± 0.2)° and (24.0 ± 0.2)°;
when measured at a temperature in the range of from 20 to 30 °C with Cu-Kalpha_{1,2} radiation having a wavelength of 0.15419 nm.

In another embodiment, the present invention relates to a crystalline solvate of tenofovir alafenamide monofumarate (form S) characterized by having a PXRD essentially the same as shown in figure 10A, 10B or 10C respectively of the present invention, when measured at a temperature in the range of from 20 to 30 °C with Cu-Kalpha_{1,2} radiation having a wavelength of 0.15419 nm.

The PXRD of form S of the present invention can be clearly distinguished from the PXRD of form I of WO 02/08241 A2 (see also PXRD overlay displayed in figure 11 herein). For instance, Form S shows the most extensive peak at (4.8 ± 0.2)° 2-Theta, whereas form I shows no reflection in the same range.

In a preferred embodiment, the crystalline solvate of tenofovir alafenamide monofumarate (form S) of the present invention is an n-propanol, n-butanol or isobutanol solvate. The solvates of the present invention are isostructural and therefore have essentially the same PXRDs (see also figures 10A, 10B and 10B).

In a further embodiment, the present invention relates to a crystalline solvate of tenofovir alafenamide monofumarate (form S) characterized by having a Raman spectrum comprising peaks at wavenumbers of:
(311 ± 2) cm⁻¹, (358 ± 2) cm⁻¹, (759 ± 2) cm⁻¹, (1199 ± 2) cm⁻¹ and (1661 ± 2) cm⁻¹; or
(311 ± 2) cm⁻¹, (358 ± 2) cm⁻¹, (759 ± 2) cm⁻¹, (1199 ± 2) cm⁻¹, (1376 ± 2) cm⁻¹, (1581 ± 2) cm⁻¹ and (1661 ± 2) cm⁻¹;
when measured at a temperature in the range of from 20 to 30 °C and a wavelength of 785 nm.

In one embodiment, a composition comprising the crystalline solvate of tenofovir alafenamide monofumarate of the present invention (form S) is essentially free of any other physical forms of tenofovir alafenamide monofumarate and/or hemifumarate. For example, a composition comprising form S of tenofovir alafenamide of the present invention comprises at most 20 weight%, preferably at most 10 weight%, more preferably at most 5 weight%, even more preferably at most 2 weight% and most preferably at most 1 weight% of any other physical form of tenofovir alafenamide monofumarate and/or hemifumarate, based on the weight of the composition.. Preferably, the any other physical form of tenofovir alafenamide monofumarate is form I of WO 02/08241 A2 and the any other physical form of tenofovir alafenamide hemifumarate is the crystalline tenofovir alafenamide hemifumarate described in WO 2013/025788 A1.

In another aspect, the present invention relates to a process for the preparation of the crystalline solvate of tenofovir alafenamide monofumarate of the present invention (form S) or the composition comprising the same as defined herein comprising:
(i) providing a solution comprising tenofovir alafenamide monofumarate and a solvent selected from the group consisting of n-propanol, n-butanol and isobutanol;
(ii) optionally seeding the solution obtained in step (i) with the crystalline solvate of tenofovir alafenamide monofumarate (form S) as defined herein;
(iii) crystallizing the solvate of tenofovir alafenamide monofumarate (form S) or the composition comprising the same as defined herein from the solution obtained in step (i) or the mixture obtained in step (ii)
(iv) optionally separating at least a part of the crystals obtained in step (iii) from their mother liquor;
(v) optionally washing the isolated crystals obtained in step (iv);
(vi)optionally drying the crystals obtained in any one of steps (iii) to (v);

Any form of tenofovir alafenamide monofumarate may be applied as starting material in the above described procedure for form S production. For example, crystalline, amorphous or mixtures of crystalline and amorphous tenofovir alafenamide monofumarate can be used. Suitable crystalline forms, which can be employed are for example form I of WO 02/08241 A2 (e.g. prepared according to reference example 1 herein), form II (e.g. prepared according to example 1 herein) or form III (e.g. prepared according to example 2 herein) of the present invention or any mixtures thereof. The nature of the applied physical form is not critical as long as the starting material is completely dissolved in the solvent upon heating and/or removed from the solution in a subsequent filtration step.

Alternatively, tenofovir alafenamide monofumarate may be prepared *in situ* by reacting tenofovir alafenamide free base with fumaric acid in the presence of a solvent comprising an alcohol selected from the group of n-propanol, n-butanol and isobutanol. Tenofovir alafenamide free base can be prepared according to the procedure disclosed in WO 02/08241 A2 (compound IV with development code GS-7340 corresponds to tenofovir alafenamide free base).

Tenofovir alafenamide monofumarate is dissolved in a solvent selected from the group of *n-*propanol, n-butanol and isobutanol upon heating to or slightly below (e.g. within 10 °C below) reflux temperature. The solvent may comprise additional organic solvents and/or water. However, most preferably n-propanol, n-butanol and isobutanol respectively is the only solvent present. The tenofovir alafenamide monofumarate concentration of the solution is preferably in the range of from about 50 to 150 g/L, more preferably from about 75 to 125 g/L and most preferably the concentration is about 100 g/L.

The hot solution is optionally filtered in order to remove any undissolved particles. Optionally, form S seed crystals are added to the hot solution in an amount ranging from about 0.1 to 20 weight%, preferably from about 1 to 10 weight% and most preferably from about 1 to 5 weight%, based on the weight of tenofovir alafenamide monofumarate.

After seeding the obtained mixture is subjected to crystallization conditions. Crystallization conditions encompass but are not limited to e.g. cooling, agitation, stirring, mixing, shaking, vibration, sonication, wet milling or any combinations thereof, which lead to the crystallization of form S. Preferably, the suspension is cooled to room temperature or below and further stirred at the applied temperature for a period in the range of from 2 to 48 hours, preferably from 4 to 24 hours and most preferably from 6 to 18 hours until crystallization is complete.

Then at least a part of the crystals are separated from their mother liquor. Preferably, the crystals are separated from their mother liquor by any conventional method such as filtration, centrifugation, solvent evaporation or decantation, more preferably by filtration or centrifugation and most preferably by filtration.

Optionally, in a further step the isolated crystals are washed with a suitable solvent, for example an organic solvent and/or water. Suitable organic solvents comprise but are not limited to alcohols such as n-propanol, n-butanol or isobutanol.

The obtained crystals may optionally be dried. Drying may be performed at a temperature of about 60 °C or less, preferably of about 40 °C or less. Typically, drying is performed at about room temperature. Drying may be performed for a period in the range of from about 1 to 72 hours, preferably from about 2 to 48 hours, more preferably from about 4 to 24 hours and most preferably from about 6 to 18 hours. Drying may be performed at ambient pressure and/ or under reduced pressure of about 100 mbar or less, more preferably of about 50 mbar or less and most preferably of about 30 mbar or less, for example a vacuum of about 20 mbar or less. Preferably, drying is performed at ambient pressure.

Tenofovir alafenamide monofumarate form S prepared according to the above described procedure may be used as seed crystals in a crystallization procedure. For example, tenofovir alafenamide monofumarate form I may be dissolved in a suitable solvent such as n-propanol, *n*-butanol or isobutanol upon heating and the obtained solution may be seeded with form S crystals in order to initiate crystallization of form S.

Therefore, in a further aspect the invention relates to the use of tenofovir alafenamide monofumarate form S of the present invention as seed crystals in a crystallization process for the preparation of tenofovir alafenamide monofumarate form S.

WO 2013/025788 A1 describes in paragraph [0071] that a "stable form screening of the monofumarate form showed that this form is not thermodynamically stable." Paragraph [0071] further states that "the monofumarate form of tenofovir alafenamide fully converts to the hemifumarate form in THF and 2-methyl THF and partially converts to the hemifumarate form in acetonitrile, ethyl acetate, MTBE and acetone (...)." It was surprisingly found by the present inventors that form I of tenofovir alafenamide monofumarate transforms to a more stable monofumarate form, namely form II of the present invention, upon slurrying for an unusual long time in acetonitrile. As can be seen from table 1, form II of the present invention possesses the higher melting point and the higher heat of fusion. Therefore, according to Burger and Ramberger's heat of fusion rule, form I and form II are monotropic with form II being the thermodynamically stable form below its melting point.

**Table 1: Comparison of DSC data**

| **DSC parameter** | **MF form I of** WO 02/08241 A2 | **MF form II of present invention** |
|---|---|---|
| Mp onset temperature | 121.2 °C | 122.3 °C |
| Heat of fusion | 100.9 J/g | 104.8 J/g |

The usage of the thermodynamically stable form is highly appreciated as polymorphic conversions, which may occur during the manufacturing process and/or during storage of a drug substance can be excluded, when the stable form is used. This ensures consistent quality and efficacy of a drug product.

In addition, the inventors of the present invention surprisingly found that in contrast to form I of WO 02/08241 A2, both form II and form III of tenofovir alafenamide monofumarate of the present invention practically show no interaction with water vapor at all. Table 2 below and figures 20A and 20B provide a comparison of the water uptakes of the different crystalline forms when subjected to moisture.

**Table 2: Water uptakes of monofumarate forms from 0 to 95% RH at (25.0 ± 0.1) °C**

| **Form** | **Water uptake from 0 to 95% RH at (25.0 ± 0.1) °C** |
|---|---|
| Form I ex WO 02/08241 A2 | 1.1 weight% |
| Form II of present invention | 0.0 weight% |
| Form III of present invention | 0.0 weight% |

The usage of non-hygroscopic forms of a compound is favorable as the physicochemical properties of such solids are preserved regardless the relative humidity of the surrounding atmosphere, which facilitates manufacturing processes as well as storage. Therefore, in a further aspect the present invention relates to the use of the crystalline form(s) II and/or III of tenofovir alafenamide monofumarate of the present invention for the preparation of a pharmaceutical composition.

The pharmaceutical composition of the present invention can be prepared by wet or dry processing methods. In certain embodiments the pharmaceutical composition is prepared by wet processing methods, such as, but not limited to, wet granulation methods. Suitable wet granulation methods comprise high-shear granulation or fluid-bed granulation. In another embodiment the pharmaceutical composition is prepared by dry processing methods, such as, but not limited to, direct compression or dry granulation methods. An example of dry granulation is roller compaction. The pharmaceutical composition obtained by dry or wet processing methods may be compressed into tablets, encapsulated or metered into sachets.

Surprisingly, the crystalline form III of tenofovir alafenamide monofumarate according to the present invention shows high physical stability, i.e. it does not convert into another polymorphic form even under increased pressure. It thus has advantageous processing properties, i.e. form III is easier to handle during tableting.

In a further aspect, the present invention relates to a pharmaceutical composition comprising an effective amount of the crystalline form(s) II and/or III of tenofovir alafenamide monofumarate, one or more pharmaceutically acceptable excipient(s) and optionally one or more additional active pharmaceutical ingredient(s).

The one or more pharmaceutically acceptable excipient(s) which is comprised in the pharmaceutical composition of the present invention is/are preferably selected from the group of carriers, fillers, diluents, lubricants, sweeteners, stabilizing agents, solubilizing agents, antioxidants and preservatives, flavouring agents, binders, colorants, osmotic agents, buffers, surfactants, disintegrants, granulating agents, coating materials and combinations thereof.

In a preferred embodiment, the pharmaceutically acceptable excipient(s) is/are selected from the group consisting of croscarmellose sodium, hydroxypropyl cellulose, lactose (as monohydrate), magnesium stearate, microcrystalline cellulose, silicon dioxide and sodium lauryl sulfate. In a preferred embodiment, all these pharmaceutically acceptable excipients are comprised by the pharmaceutical composition of the present invention.

In one embodiment, the one or more additional active pharmaceutical ingredient(s) is/are selected from the group of elvitegravir, cobicistat, emtricitabine, darunavir and rilpivirine.

The pharmaceutical composition of the present invention is preferably an oral solid dosage form, such as a tablet or a capsule. Preferably, the pharmaceutical composition of the present invention is a tablet. In a preferred embodiment, the tablet is film-coated with a coating material containing FD&C Blue No. 2/indigo carmine aluminum lake, iron oxide yellow, polyethylene glycol, polyvinyl alcohol, talc, and titanium dioxide.

Preferably, the present invention relates to a pharmaceutical composition as describe above, wherein the effective amount of the crystalline form(s) II and/or III of tenofovir alafenamide monofumarate is about 25 mg, calculated as tenofovir alafenamide. The crystalline form(s) II and/or III of tenofovir alafenamide may be used in amounts of 3 mg, 8 ± 3mg, 10 ± 5 mg, 25 ± 5 mg, or 40 ± 10 mg or other ranges (calculated as tenofovir alafenamide).

Preferably, the present invention relates to a pharmaceutical composition as described above, wherein the pharmaceutical composition is to be administered once-daily.

In another embodiment, the invention provides an anti-virus agent(s) comprising (a) the crystalline form(s) II and/or III of tenofovir alafenamide monofumarate and (b) cobicistat, or a pharmaceutically acceptable salt thereof, in combination. The anti-virus agent(s) may include cobicistat in amounts of 50-500 mg, 100-400 mg, 100-300 mg or 150 mg. The anti-virus agent may further include 200 mg of emtricitabine and 150 mg of elvitegravir. The anti-virus agent may include the crystalline form(s) II and/or III of tenofovir alafenamide monofumarate in amounts as described above, 150 mg cobicistat, 150 mg elvitegravir, and 200 mg emtricitabine. Suitable formulations for the crystalline form(s) II and/or III of tenofovir alafenamide monofumarate include those described in WO 2010/091197, which is incorporated by reference, with the proviso that whenever WO 2010/091197 refers to "a compound of Formula IV (i.e. tenofovir disoproxil fumarate)", it should be replaced by an effective amount of the crystalline form II and/or III of tenofovir alafenamide monofumarate.

In a further aspect, the present invention relates to the pharmaceutical composition as described above for use as a medicament.

In yet another aspect, the present invention relates to the pharmaceutical composition as described above for use in the treatment or prophylaxis of viral infections caused by DNA viruses, RNA viruses, herpesviruses (e.g. CMV, HSV 1, HSV 2, VZV), retroviruses, hepadnaviruses (e.g. HBV), papillomavirus, hantavirus, adenoviruses and HIV.

In a particular embodiment, the present invention relates to the crystalline form(s) II and/or III of tenofovir alafenamide monofumarate or pharmaceutical compositions as described above for use in the treatment or prophylaxis of HIV-1 infections.

In a further particular embodiment, the present invention relates to the crystalline form(s) II and/or III of tenofovir alafenamide monofumarate or pharmaceutical compositions as described above for use in the treatment or prophylaxis of Hepatitis B (HBV) infections.

The crystalline form(s) II and/or III of tenofovir alafenamide monofumarate may also be used in the following combinations (including, but not limited to single tablet regimens):
(a) emtricitabine / darunavir / cobicistat / tenofovir alafenamide monofumarate;
(b) emtricitabine / elvitegravir / cobicistat / tenofovir alafenamide monofumarate;
(c) cobicistat / tenofovir alafenamide monofumarate;
(d) emtricitabine / tenofovir alafenamide monofumarate;
(e) GS-9883 / emtricitabine / tenofovir alafenamide monofumarate;
(f) rilpivirine / emtricitabine / tenofovir alafenamide monofumarate;
(g) efavirenz / emtricitabine / tenofovir alafenamide monofumarate.

The combinations listed above may contain various dosages of the component agents.

### Embodiment section

Aspects, advantageous features and preferred embodiments of the present invention are summarized in the following items:
1) A crystalline form of tenofovir alafenamide monofumarate (form II) characterized by having a powder X-ray diffractogram comprising reflections at 2-Theta angles of (7.3 ± 0.2)°, (9.4 ± 0.1)° and (10.1 ± 0.1)°, when measured at a temperature in the range of from 20 to 30 °C with CuKalpha_{1,2} radiation having a wavelength of 0.15419 nm.
2) A crystalline form of tenofovir alafenamide monofumarate (form II) characterized by having a powder X-ray diffractogram comprising reflections at 2-Theta angles of (7.3 ± 0.2)°, (9.4 ± 0.1)°, (10.1 ± 0.1)° and (13.0 ± 0.1)°, when measured at a temperature in the range of from 20 to 30 °C with CuKalpha_{1,2} radiation having a wavelength of 0.15419 nm.
3) A crystalline form of tenofovir alafenamide monofumarate (form II) characterized by having a powder X-ray diffractogram comprising reflections at 2-Theta angles of (5.6 ± 0.1)°, (7.3 ± 0.2)°, (9.4 ± 0.1)°, (10.1 ± 0.1)° and (13.0 ± 0.1)°, when measured at a temperature in the range of from 20 to 30 °C with CuKalpha_{1,2} radiation having a wavelength of 0.15419 nm.
4) A crystalline form of tenofovir alafenamide monofumarate (form II) characterized by having a powder X-ray diffractogram comprising reflections at 2-Theta angles of (5.6 ± 0.1)°, (7.3 ± 0.2)°, (9.4 ± 0.1)°, (10.1 ± 0.1)°, (13.0 ± 0.1)° and (28.4 ± 0.1)°, when measured at a temperature in the range of from 20 to 30 °C with CuKalpha_{1,2} radiation having a wavelength of 0.15419 nm.
5) A crystalline form of tenofovir alafenamide monofumarate (form II) characterized by having a powder X-ray diffractogram essentially the same as shown in figure 1 of the present invention, when measured at a temperature in the range of from 20 to 30 °C with Cu-Kalpha_{1,2} radiation having a wavelength of 0.15419 nm.
6) The crystalline form according to any one of the preceding items characterized by showing an endotherm with an onset temperature in the range of from 122.0 to 122.5 °C, when measured with differential scanning calorimetry at a heating rate of 10 K/min.
7) The crystalline form according to any one of items 1 to 5 characterized by showing an endotherm with an onset temperature of 122 °C, when measured with differential scanning calorimetry at a heating rate of 10 K/min.
8) The crystalline form according to any one of items 1 to 5 characterized by showing an endotherm with an onset temperature of 122.3 °C, when measured with differential scanning calorimetry at a heating rate of 10 K/min.
9) The crystalline form according to any one of the preceding items characterized by having a heat of fusion of at least 102.0 J/g, when measured with differential scanning calorimetry at a heating rate of 10 K/min.
10) The crystalline form according to any one of items 1 to 8, characterized by having a heat of fusion of at least 103.0 J/g, when measured with differential scanning calorimetry at a heating rate of 10 K/min.
11) The crystalline form according to any one of items 1 to 8, characterized by having a heat of fusion of at least 104.0 J/g, when measured with differential scanning calorimetry at a heating rate of 10 K/min.
12) The crystalline form according to any one of items 1 to 8, characterized by having a heat of fusion of at least 104.5 J/g, when measured with DSC at a heating rate of 10 K/min.
13) The crystalline form according to any one of items 1 to 8, characterized by having a heat of fusion of 104.8 J/g, when measured with DSC at a heating rate of 10 K/min.
14) The crystalline form according to any one of the preceding items characterized by showing a mass loss of not more than 2.0 weight%, based on the weight of the crystalline form, when measured with thermogravimetric analysis at a temperature in the range of from 25 to 130 °C and a heating rate of 10 K/min.
15) The crystalline form according to any one of items 1 to 13 characterized by showing a mass loss of not more than 1.5 weight%, based on the weight of the crystalline form, when measured with thermogravimetric analysis at a temperature in the range of from 25 to 130 °C and a heating rate of 10 K/min.
16) The crystalline form according to any one of items 1 to 13 characterized by showing a mass loss of not more than 1.0 weight%, based on the weight of the crystalline form, when measured with thermogravimetric analysis at a temperature in the range of from 25 to 130 °C and a heating rate of 10 K/min.
17) The crystalline form according to any one of items 1 to 13 characterized by showing a mass loss of not more than 0.5 weight%, based on the weight of the crystalline form, when measured with thermogravimetric analysis at a temperature in the range of from 25 to 130 °C and a heating rate of 10 K/min.
18) The crystalline form according to any one of items 1 to 13 characterized by showing a mass loss of not more than 0.3 weight%, based on the weight of the crystalline form, when measured with thermogravimetric analysis at a temperature in the range of from 25 to 130 °C and a heating rate of 10 K/min.
19) The crystalline form according to any one of the preceding items characterized by showing a mass change of not more than 2.0 weight%, based on the weight of the crystalline form, when measured with gravimetric moisture sorption at a relative humidity in the range of from 0 to 95% and a temperature of (25.0 ± 0.1)°C.
20) The crystalline form according to any one of items 1 to 18 characterized by showing a mass change of not more than 1.5 weight%, based on the weight of the crystalline form, when measured with gravimetric moisture sorption at a relative humidity in the range of from 0 to 95% and a temperature of (25.0 ± 0.1)°C.
21) The crystalline form according to any one of items 1 to 18 characterized by showing a mass change of not more than 1.0 weight% based on the weight of the crystalline form, when measured with gravimetric moisture sorption at a relative humidity in the range of from 0 to 95% and a temperature of (25.0 ± 0.1)°C.
22) The crystalline form according to any one of items 1 to 18 characterized by showing a mass change of not more than 0.5 weight%, based on the weight of the crystalline form, when measured with gravimetric moisture sorption at a relative humidity in the range of from 0 to 95% and a temperature of (25.0 ± 0.1)°C.
23) The crystalline form according to any one of items 1 to 18 characterized by showing a mass change of not more than 0.4 weight%, based on the weight of the crystalline form, when measured with gravimetric moisture sorption at a relative humidity in the range of from 0 to 95% and a temperature of (25.0 ± 0.1)°C.
24) The crystalline form according to any one of items 1 to 18 characterized by showing a mass change of not more than 0.3 weight%, based on the weight of the crystalline form, when measured with gravimetric moisture sorption at a relative humidity in the range of from 0 to 95% and a temperature of (25.0 ± 0.1)°C.
25) The crystalline form according to any one of items 1 to 18 characterized by showing a mass change of not more than 0.2 weight%, based on the weight of the crystalline form, when measured with gravimetric moisture sorption at a relative humidity in the range of from 0 to 95% and a temperature of (25.0 ± 0.1)°C.
26) The crystalline form according to any one of items 1 to 18 characterized by showing a mass change of not more than 0.1 weight%, based on the weight of the crystalline form, when measured with gravimetric moisture sorption at a relative humidity in the range of from 0 to 95% and a temperature of (25.0 ± 0.1)°C.
27) The crystalline form of tenofovir alafenamide monofumarate according to any one of the preceding items characterized by having a Raman spectrum comprising peaks at wavenumbers of (347 ± 2) cm⁻¹, (477 ± 2) cm⁻¹, (758 ± 2) cm⁻¹, (863 ± 2) cm⁻¹ and (1244 ± 2) cm⁻¹, when measured at a temperature in the range of from 20 to 30 °C and a wavelength of 785 nm.
28) The crystalline form of tenofovir alafenamide monofumarate according to any one of items 1 to 26 characterized by having a Raman spectrum comprising peaks at wavenumbers of (347 ± 2) cm⁻¹, (400 ± 2) cm⁻¹, (477 ± 2) cm⁻¹, (561 ± 2) cm⁻¹, (758 ± 2) cm⁻¹, (863 ± 2) cm⁻¹, (878 ± 2) cm⁻¹ and (1244 ± 2) cm⁻¹, when measured at a temperature in the range of from 20 to 30 °C and a wavelength of 785 nm.
29) The crystalline form of tenofovir alafenamide monofumarate according to any one of items 1 to 26 characterized by having a Raman spectrum comprising peaks at wavenumbers of (347 ± 2) cm⁻¹, (400 ± 2) cm⁻¹, (477 ± 2) cm⁻¹, (561 ± 2) cm⁻¹, (758 ± 2) cm⁻¹, (863 ± 2) cm⁻¹, (878 ± 2) cm⁻¹, (1089 ± 2) cm⁻¹, (1151 ± 2) cm⁻¹, (1244 ± 2) cm⁻¹, (1395 ± 2) cm⁻¹, (1419 ± 2) cm⁻¹ and (1442 ± 2) cm⁻¹, when measured at a temperature in the range of from 20 to 30 °C and a wavelength of 785 nm.
30) A composition comprising the crystalline form according to any one of the preceding items, which composition is essentially free of any other physical form of tenofovir alafenamide monofumarate and/or hemifumarate.
31) A composition comprising the crystalline form according to any one of items 1 to 29, characterized by comprising at most 20 weight% of any other physical form of tenofovir alafenamide monofumarate and/or hemifumarate, based on the weight of the composition.
32) A composition comprising the crystalline form according to any one of items 1 to 29, characterized by comprising at most 10 weight% of any other physical form of tenofovir alafenamide monofumarate and/or hemifumarate, based on the weight of the composition.
33) A composition comprising the crystalline form according to any one of items 1 to 29, characterized by comprising at most 5 weight% of any other physical form of tenofovir alafenamide monofumarate and/or hemifumarate, based on the weight of the composition.
34) A composition comprising the crystalline form according to any one of items 1 to 29, characterized by comprising at most 2 weight% of any other physical form of tenofovir alafenamide monofumarate and/or hemifumarate, based on the weight of the composition.
35) A composition comprising the crystalline form according to any one of items 1 to 29, characterized by comprising at most 1 weight% of any other physical form of tenofovir alafenamide monofumarate and/or hemifumarate, based on the weight of the composition.
36) The composition according to any one of items 30 to 35, wherein the any other physical form of tenofovir alafenamide monofumarate is characterized by having a powder X-ray diffractogram comprising reflections at 2-Theta angles of (5.3 ± 0.1)°, (9.8 ± 0.1)°, (10.4 ± 0.1)°, (15.9 ± 0.1)°, (16.2 ± 0.1)° and (16.6 ± 0.1)°, when measured at a temperature in the range of from 20 to 30 °C with Cu-Kalpha_{1,2} radiation having a wavelength of 0.15419 nm.
37) The composition according to any one of items 30 to 35, wherein the any other physical form of tenofovir alafenamide hemifumarate is characterized by having a powder X-ray diffractogram comprising reflections at 2-Theta angles of (6.9 ± 0.2)°, (8.6 ± 0.2)°, (10.0 ± 0.2)°, (11.0 ± 0.2)°, (12.2 ± 0.2)°, (15.9 ± 0.2)°, (16.3 ± 0.2)°, (20.2 ± 0.2)° and (20.8 ± 0.2)°, when measured at a temperature in the range of from 20 to 30 °C with Cu-Kalpha_{1,2} radiation having a wavelength of 0.15419 nm.
38) A process for the preparation of the crystalline form as defined in any one of items 1 to 29 or the composition as defined in any one of items 30 to 37 comprising:
   (i) providing tenofovir alafenamide monofumarate in solid form;
   (ii) slurrying tenofovir alafenamide monofumarate provided in step (i) in a solvent comprising acetonitrile for at least 10 days;
39) The process of item 38, wherein the solid form in step (i) is tenofovir alafenamide monofumarate characterized by having a powder X-ray diffractogram comprising reflections at 2-Theta angles of (5.3 ± 0.1)°, (9.8 ± 0.1)°, (10.4 ± 0.1)°, (15.9 ± 0.1)°, (16.2 ± 0.1)° and (16.6 ± 0.1)°, when measured at a temperature in the range of from 20 to 30 °C with Cu-Kalpha_{1,2} radiation having a wavelength of 0.15419 nm.
40) The process of item 38 or 39, wherein acetonitrile is the only solvent present in the slurry provided in step (ii).
41) The process according to any one of items 38 to 40, wherein the tenofovir alafenamide monofumarate concentration of the suspension provided in step (ii) is in the range of from 10 to 200 g/L.
42) The process according to any one of items 38 to 40, wherein the tenofovir alafenamide monofumarate concentration of the suspension provided in step (ii) is in the range of from 50 to 150 g/L.
43) The process according to any one of items 38 to 40, wherein the tenofovir alafenamide monofumarate concentration of the suspension provided in step (ii) is in the range of from 75 to 125 g/L.
44) The process according to any one of items 38 to 40, wherein the tenofovir alafenamide monofumarate concentration of the suspension provided in step (ii) is 100 g/L.
45) The process according to any one of items 38 to 44, wherein slurrying is performed at a temperature in the range of from 20 to 30 °C.
46) The process according to any one of items 38 to 44, wherein slurrying is performed at a temperature in the range of from 40 to 80 °C.
47) The process according to any one of items 38 to 44, wherein slurrying is performed at a temperature in the range of from 40 to 60 °C.
48) The process according to any one of items 38 to 47, further comprising step (iii) separating at least a part of the crystals obtained in step (ii) from its mother liquor.
49) The process according to item 48, wherein the crystals are separated from their mother liquor by filtration, centrifugation, decantation or solvent evaporation.
50) The process according to item 48, wherein the crystals are separated from their mother liquor by filtration or centrifugation.
51) The process according to item 48, wherein the crystals are separated from their mother liquor by filtration.
52) The process according to any one of items 48 to 51, further comprising step (iv) washing the isolated crystals obtained in step (iii).
53) The process according to item 52, wherein the crystals are washed with an organic solvent and/or water.
54) The process according to item 53, wherein the organic solvent comprises acetonitrile.
55) The process according to any one of items 38 to 54 further comprising drying the crystals obtained in any one of steps (ii) to (iv).
56) The process according to item 55, wherein drying is performed at a temperature of 100 °C or less.
57) The process according to item 55, wherein drying is performed at a temperature of 80 °C or less.
58) The process according to item 55, wherein drying is performed at a temperature of 60 °C or less.
59) The process according to item 55, wherein drying is performed at a temperature of 40 °C or less.
60) The process according to item 55, wherein drying is performed at a temperature in the range of from 20 to 30 °C.
61) The process according to any one of items 55 to 60, wherein drying is performed for a period in the range of from 1 to 72 hours.
62) The process according to any one of items 55 to 60, wherein drying is performed for a period in the range of from 2 to 48 hours.
63) The process according to any one of items 55 to 60, wherein drying is performed for a period in the range of from 4 to 24 hours.
64) The process according to any one of items 55 to 60, wherein drying is performed for a period in the range of from 6 to 18 hours.
65) Use of the crystalline form as defined in any one of items 1 to 29 or the composition as defined in any one of items 30 to 37 or as obtained from the process according to any one of items 38 to 64 as seed crystals in a crystallization process for the preparation of the crystalline form as defined in any one of items 1 to 29 or the composition according to any one of items 30 to 37.
66) Use of the crystalline form as defined in any one of items 1 to 29 or the composition as defined in any one of items 30 to 37 for the preparation of a pharmaceutical composition.
67) The use according to item 66, wherein the pharmaceutical composition is prepared by a wet or dry processing method.
68) The use according to item 67, wherein the wet processing method comprises wet granulation.
69) The use according to item 67, wherein the dry processing method comprises dry granulation or dry compaction.
70) A pharmaceutical composition comprising an effective amount of the crystalline form as defined in any one of items 1 to 29 or the composition as defined in any one of items 30 to 37 and one or more pharmaceutically acceptable excipient(s).
71) The pharmaceutical composition of item 70, wherein the one or more pharmaceutically acceptable excipient(s) is/are selected from the group of carriers, fillers, diluents, lubricants, sweeteners, stabilizing agents, solubilizing agents, antioxidants and preservatives, flavouring agents, binders, colorants, osmotic agents, buffers, surfactants, disintegrants, granulating agents, coating materials and combinations thereof.
72) The pharmaceutical composition according to item 70 or 71 comprising one or more additional active pharmaceutical ingredient(s).
73) The pharmaceutical composition according to item 72, wherein the one or more additional pharmaceutical active ingredient(s) is/are selected from the group consisting of elvitegravir, cobicistat, emtricitabine, darunavir and rilpivirine.
74) The pharmaceutical composition according to any one of items 70 to 73 which is an oral solid dosage form.
75) The pharmaceutical composition according to item 74, wherein the oral solid dosage form is a tablet or a capsule.
76) The pharmaceutical composition according to any one of items 70 to 75 comprising 25 mg of the crystalline form as defined in any one of items 1 to 29, calculated as tenofovir alafenamide.
77) The pharmaceutical composition according to any one of items 70 to 76, which is administered once daily.
78) The pharmaceutical composition according to any one of items 70 to 77 for use as a medicament.
79) The pharmaceutical composition according to any one of items 70 to 77 for use in the treatment and/or prophylaxis of viral infections.
80) The pharmaceutical composition according to item 79, wherein the viral infection is caused by DNA viruses, RNA viruses, herpesviruses, retroviruses, hepadnaviruses, papillomavirus, hantavirus, adenoviruses and HIV.
81) A crystalline form of tenofovir alafenamide monofumarate (form III) characterized by having a powder X-ray diffractogram comprising reflections at 2-Theta angles of (5.6 ± 0.1)°, (7.3 ± 0.2)° and (10.5 ± 0.1)°, when measured at a temperature in the range of from 20 to 30 °C with CuKalpha_{1,2} radiation having a wavelength of 0.15419 nm.
82) A crystalline form of tenofovir alafenamide monofumarate (form III) characterized by having a powder X-ray diffractogram comprising reflections at 2-Theta angles of (5.6 ± 0.1)°, (7.3 ± 0.2)°, (10.5 ± 0.1)° and (12.6 ± 0.1)°, when measured at a temperature in the range of from 20 to 30 °C with CuKalpha_{1,2} radiation having a wavelength of 0.15419 nm.
83) A crystalline form of tenofovir alafenamide monofumarate (form III) characterized by having a powder X-ray diffractogram comprising reflections at 2-Theta angles of (5.6 ± 0.1)°, (7.3 ± 0.2)°, (10.5 ± 0.1)°, (12.6 ± 0.1)° and (17.0 ± 0.1)°, when measured at a temperature in the range of from 20 to 30 °C with CuKalpha_{1,2} radiation having a wavelength of 0.15419 nm.
84) A crystalline form of tenofovir alafenamide monofumarate (form III) characterized by having a powder X-ray diffractogram comprising reflections at 2-Theta angles of (5.6 ± 0.1)°, (7.3 ± 0.2)°, (9.4 ± 0.1)°, (10.5 ± 0.1)°, (12.6 ± 0.1)° and (17.0 ± 0.1)°, when measured at a temperature in the range of from 20 to 30 °C with CuKalpha_{1,2} radiation having a wavelength of 0.15419 nm.
85) A crystalline form of tenofovir alafenamide monofumarate (form III) characterized by having a powder X-ray diffractogram essentially the same as shown in figure 6 of the present invention, when measured at a temperature in the range of from 20 to 30 °C with Cu-Kalpha_{1,2} radiation having a wavelength of 0.15419 nm.
86) The crystalline form according to any one of items 81 to 85 characterized by showing a mass loss of not more than 2.0 weight%, based on the weight of the crystalline form, when measured with thermogravimetric analysis at a temperature in the range of from 25 to 130 °C and a heating rate of 10 K/min.
87) The crystalline form according to any one of items 81 to 85 characterized by showing a mass loss of not more than 1.5 weight%, based on the weight of the crystalline form, when measured with thermogravimetric analysis at a temperature in the range of from 25 to 130 °C and a heating rate of 10 K/min.
88) The crystalline form according to any one of items 81 to 85 characterized by showing a mass loss of not more than 1.0 weight%, based on the weight of the crystalline form, when measured with thermogravimetric analysis at a temperature in the range of from 25 to 130 °C and a heating rate of 10 K/min.
89) The crystalline form according to any one of items 81 to 85 characterized by showing a mass loss of not more than 0.5 weight%, based on the weight of the crystalline form, when measured with thermogravimetric analysis at a temperature in the range of from 25 to 130 °C and a heating rate of 10 K/min.
90) The crystalline form according to any one of items 81 to 85 characterized by showing a mass loss of not more than 0.4 weight%, based on the weight of the crystalline form, when measured with thermogravimetric analysis at a temperature in the range of from 25 to 130 °C and a heating rate of 10 K/min.
91) The crystalline form according to any one of items 81 to 85 characterized by showing a mass loss of not more than 0.3 weight%, based on the weight of the crystalline form, when measured with thermogravimetric analysis at a temperature in the range of from 25 to 130 °C and a heating rate of 10 K/min.
92) The crystalline form according to any one of items 81 to 85 characterized by showing a mass loss of not more than 0.2 weight%, based on the weight of the crystalline form, when measured with thermogravimetric analysis at a temperature in the range of from 25 to 130 °C and a heating rate of 10 K/min.
93) The crystalline form according to any one of items 81 to 85 characterized by showing a mass loss of not more than 0.1 weight%, based on the weight of the crystalline form, when measured with thermogravimetric analysis at a temperature in the range of from 25 to 130 °C and a heating rate of 10 K/min.
94) The crystalline form according to any one of items 81 to 93 characterized by showing a mass change of not more than 2.0 weight%, based on the weight of the crystalline form, when measured with gravimetric moisture sorption at a relative humidity in the range of from 0 to 95% and a temperature of (25.0 ± 0.1) °C.
95) The crystalline form according to any one of items 81 to 93 characterized by showing a mass change of not more than 1.5 weight%, based on the weight of the crystalline form, when measured with gravimetric moisture sorption at a relative humidity in the range of from 0 to 95% and a temperature of (25.0 ± 0.1) °C.
96) The crystalline form according to any one of items 81 to 93 characterized by showing a mass change of not more than 1.0 weight% based on the weight of the crystalline form, when measured with gravimetric moisture sorption at a relative humidity in the range of from 0 to 95% and a temperature of (25.0 ± 0.1) °C.
97) The crystalline form according to any one of items 81 to 93 characterized by showing a mass change of not more than 0.5 weight%, based on the weight of the crystalline form, when measured with gravimetric moisture sorption at a relative humidity in the range of from 0 to 95% and a temperature of (25.0 ± 0.1)°C.
98) The crystalline form according to any one of items 81 to 93 characterized by showing a mass change of not more than 0.4 weight%, based on the weight of the crystalline form, when measured with gravimetric moisture sorption at a relative humidity in the range of from 0 to 95% and a temperature of (25.0 ± 0.1)°C.
99) The crystalline form according to any one of items 81 to 93 characterized by showing a mass change of not more than 0.3 weight%, based on the weight of the crystalline form, when measured with gravimetric moisture sorption at a relative humidity in the range of from 0 to 95% and a temperature of (25.0 ± 0.1)°C.
100) The crystalline form according to any one of items 81 to 93 characterized by showing a mass change of not more than 0.2 weight%, based on the weight of the crystalline form, when measured with gravimetric moisture sorption at a relative humidity in the range of from 0 to 95% and a temperature of (25.0 ± 0.1)°C.
101) The crystalline form according to any one of items 81 to 93 characterized by showing a mass change of not more than 0.1 weight%, based on the weight of the crystalline form, when measured with gravimetric moisture sorption at a relative humidity in the range of from 0 to 95% and a temperature of (25.0 ± 0.1)°C.
102) The crystalline form according to any one of items 81 to 101 characterized by having a Raman spectrum comprising peaks at wavenumbers of (348 ± 2) cm⁻¹, (478 ± 2) cm⁻¹, (756 ± 2) cm⁻¹, (862 ± 2) cm⁻¹ and (1256 ± 2) cm⁻¹, when measured at a temperature in the range of from 20 to 30 °C and a wavelength of 785 nm.
103) The crystalline form according to any one of items 81 to 101 characterized by having a Raman spectrum comprising peaks at wavenumbers of (348 ± 2) cm⁻¹, (400 ± 2) cm⁻¹, (478 ± 2) cm⁻¹, (563 ± 2) cm⁻¹, (756 ± 2) cm⁻¹, (862 ± 2) cm⁻¹, (1089 ± 2) cm⁻¹ and (1256 ± 2) cm⁻¹, when measured at a temperature in the range of from 20 to 30 °C and a wavelength of 785 nm.
104) The crystalline form according to any one of items 81 to 101 characterized by having a Raman spectrum comprising peaks at wavenumbers of (348 ± 2) cm⁻¹, (400 ± 2) cm⁻¹, (478 ± 2) cm⁻¹, (563 ± 2) cm⁻¹, (756 ± 2) cm⁻¹, (801 ± 2) cm⁻¹, (862 ± 2) cm⁻¹, (1089 ± 2) cm⁻¹, (1151 ± 2) cm⁻¹, (1256 ± 2) cm⁻¹, (1397 ± 2) cm⁻¹, (1419 ± 2) cm⁻¹ and (1442 ± 2) cm⁻¹, when measured at a temperature in the range of from 20 to 30 °C and a wavelength of 785 nm.
105) A composition comprising the crystalline form according to any one of items 81 to 104, which composition is essentially free of any other physical form of tenofovir alafenamide monofumarate and/or hemifumarate.
106) A composition comprising the crystalline form according to any one of items 81 to 104, characterized by comprising at most 20 weight% of any other physical form of tenofovir alafenamide monofumarate and/or hemifumarate, based on the weight of the composition.
107) A composition comprising the crystalline form according to any one of items 81 to 104, characterized by comprising at most 10 weight% of any other physical form of tenofovir alafenamide monofumarate and/or hemifumarate, based on the weight of the composition.
108) A composition comprising the crystalline form according to any one of items 81 to 104, characterized by comprising at most 5 weight% of any other physical form of tenofovir alafenamide monofumarate and/or hemifumarate, based on the weight of the composition.
109) A composition comprising the crystalline form according to any one of items 81 to 104, characterized by comprising at most 2 weight% of any other physical form of tenofovir alafenamide monofumarate and/or hemifumarate, based on the weight of the composition.
110) A composition comprising the crystalline form according to any one of items 81 to 104, characterized by comprising at most 1 weight% of any other physical form of tenofovir alafenamide monofumarate and/or hemifumarate, based on the weight of the composition.
111) The composition according to any one of items 105 to 110, wherein the any other physical form of tenofovir alafenamide monofumarate is characterized by having a powder X-ray diffractogram comprising reflections at 2-Theta angles of (5.3 ± 0.1)°, (9.8 ± 0.1)°, (10.4 ± 0.1)°, (15.9 ± 0.1)°, (16.2 ± 0.1)° and (16.6 ± 0.1)°, when measured at a temperature in the range of from 20 to 30 °C with Cu-Kalpha_{1,2} radiation having a wavelength of 0.15419 nm.
112) The composition according to any one of items 105 to 110, wherein the any other physical form of tenofovir alafenamide hemifumarate is characterized by having a powder X-ray diffractogram comprising reflections at 2-Theta angles of (6.9 ± 0.2)°, (8.6 ± 0.2)°, (10.0 ± 0.2)°, (11.0 ± 0.2)°, (12.2 ± 0.2)°, (15.9 ± 0.2)°, (16.3 ± 0.2)°, (20.2 ± 0.2)° and (20.8 ± 0.2)°, when measured at a temperature in the range of from 20 to 30 °C with Cu-Kalpha_{1,2} radiation having a wavelength of 0.15419 nm.
113) A process for the preparation of the crystalline form as defined in any one of items 81 to 104 or the composition as defined in any one of items 105 to 112 comprising:
   (i) providing a crystalline solvate of tenofovir alafenamide monofumarate (form S) as defined in any one of items 140 to 149;
   (ii) at least partially desolvating the crystalline solvate provided in step (i) at a temperature in the range of from 100 to 115 °C;
114) The process of item 113, wherein the desolvation in step (ii) is performed at a temperature of at least 100 °C, with the proviso that the temperature does not exceed 115 °C.
115) The process of item 113, wherein the desolvation in step (ii) is performed at a temperature of at least 105 °C, with the proviso that the temperature does not exceed 115 °C.
116) The process of item 113, wherein the desolvation in step (ii) is performed at a temperature of at least 110 °C, with the proviso that the temperature does not exceed 115 °C.
117) The process according to any one of items 113 to 116, wherein the desolvation in step (ii) is performed by applying a vacuum of 100 mbar or less.
118) The process according to any one of items 113 to 116, wherein the desolvation in step (ii) is performed by applying a vacuum of 50 mbar or less.
119) The process according to any one of items 113 to 116, wherein the desolvation in step (ii) is performed by applying a vacuum of 30 mbar or less.
120) The process according to any one of items 113 to 116, wherein the desolvation in step (ii) is performed by applying a vacuum of 20 mbar or less.
121) The process according to any one of items 113 to 120, wherein the desolvation is performed for a period in the range of from 1 to 72 hours.
122) The process according to any one of items 113 to 120, wherein the desolvation is performed for a period in the range of from 2 to 48 hours.
123) The process according to any one of items 113 to 120, wherein the desolvation is performed for a period in the range of from 4 to 24 hours.
124) The process according to any one of items 113 to 120, wherein the desolvation is performed for a period in the range of from 4 to 12 hours.
125) Use of the crystalline form as defined in any one of items 81 to 104 or the composition as defined in any one of items 105 to 112 or as obtained from the process according to any one of items 113 to 124 for the preparation of a pharmaceutical composition.
126) The use according to item 125, wherein the pharmaceutical composition is prepared by a wet or dry processing method.
127) The use according to item 126, wherein the wet processing method comprises wet granulation.
128) The use according to item 126, wherein the dry processing method comprises dry granulation or dry compaction.
129) A pharmaceutical composition comprising an effective amount of the crystalline form as defined in any one of items 81 to 104 or the composition as defined in any one of items 105 to 112 and one or more pharmaceutically acceptable excipient(s).
130) The pharmaceutical composition of item 129, wherein the one or more pharmaceutically acceptable excipient(s) is/are selected from the group of carriers, fillers, diluents, lubricants, sweeteners, stabilizing agents, solubilizing agents, antioxidants and preservatives, flavouring agents, binders, colorants, osmotic agents, buffers, surfactants, disintegrants, granulating agents, coating materials and combinations thereof.
131) The pharmaceutical composition according to item 129 or 130 comprising one or more additional active pharmaceutical ingredient(s).
132) The pharmaceutical composition according to item 131, wherein the one or more additional pharmaceutical active ingredient(s) is/are selected from the group consisting of elvitegravir, cobicistat, emtricitabine, darunavir and rilpivirine.
133) The pharmaceutical composition according to any one of items 129 to 132 which is an oral solid dosage form.
134) The pharmaceutical composition according to item 133, wherein the oral solid dosage form is a tablet or a capsule.
135) The pharmaceutical composition according to any one of items 129 to 134 comprising 25 mg of the crystalline form as defined in any one of items 81 to 104, calculated as tenofovir alafenamide.
136) The pharmaceutical composition according to any one of items 129 to 135, which is administered once daily.
137) The pharmaceutical composition according to any one of items 129 to 136 for use as a medicament.
138) The pharmaceutical composition according to any one of items 129 to 136 for use in the treatment and/or prophylaxis of viral infections.
139) The pharmaceutical composition according to item 138, wherein the viral infection is caused by DNA viruses, RNA viruses, herpesviruses, retroviruses, hepadnaviruses, papillomavirus, hantavirus, adenoviruses and HIV.
140) A crystalline solvate of tenofovir alafenamide monofumarate (form S) characterized by having a powder X-ray diffractogram comprising reflections at 2-Theta angles of (4.8 ± 0.2)°, (10.3 ± 0.2)° and (10.6 ± 0.2)°, when measured at a temperature in the range of from 20 to 30 °C with CuKalpha_{1,2} radiation having a wavelength of 0.15419 nm.
141) A crystalline solvate of tenofovir alafenamide monofumarate (form S) characterized by having a powder X-ray diffractogram comprising reflections at 2-Theta angles of (4.8 ± 0.2)°, (10.3 ± 0.2)°, (10.6 ± 0.2)° and (24.0 ± 0.2)°, when measured at a temperature in the range of from 20 to 30 °C with CuKalpha_{1,2} radiation having a wavelength of 0.15419 nm.
142) A crystalline solvate of tenofovir alafenamide monofumarate (form S) characterized by having a powder X-ray diffractogram comprising reflections at 2-Theta angles of (4.8 ± 0.2)°, (10.3 ± 0.2)°, (10.6 ± 0.2)°, (15.1 ± 0.2)° and (24.0 ± 0.2)°, when measured at a temperature in the range of from 20 to 30 °C with CuKalpha_{1,2} radiation having a wavelength of 0.15419 nm.
143) A crystalline solvate of tenofovir alafenamide monofumarate (form S) characterized by having a powder X-ray diffractogram comprising reflections at 2-Theta angles of (4.8 ± 0.2)°, (10.3 ± 0.2)°, (10.6 ± 0.2)°, (15.1 ± 0.2)°, (15.3 ± 0.2)° and (24.0 ± 0.2)°, when measured at a temperature in the range of from 20 to 30 °C with CuKalpha_{1,2} radiation having a wavelength of 0.15419 nm.
144) A crystalline solvate of tenofovir alafenamide monofumarate (form S) characterized by having a powder X-ray diffractogram essentially the same as shown in any one of figures 10A, 10B or 10C of the present invention, when measured at a temperature in the range of from 20 to 30 °C with Cu-Kalpha_{1,2} radiation having a wavelength of 0.15419 nm.
145) The crystalline solvate according to any one of items 140 to 144, which is an n-propanol solvate.
146) The crystalline solvate according to any one of items 140 to 144, which is an n-butanol solvate.
147) The crystalline solvate according to any one of items 140 to 144, which is an isobutanol solvate.
148) The crystalline solvate of tenofovir alafenamide monofumarate according to any one of items 140 to 147 characterized by having a Raman spectrum comprising peaks at wavenumbers of (311 ± 2) cm⁻¹, (358 ± 2) cm⁻¹, (759 ± 2) cm⁻¹, (1199 ± 2) cm⁻¹ and (1661 ± 2) cm⁻¹; when measured at a temperature in the range of from 20 to 30 °C and a wavelength of 785 nm.
149) The crystalline solvate of tenofovir alafenamide monofumarate according to any one of items 140 to 147 characterized by having a Raman spectrum comprising peaks at wavenumbers of (311 ± 2) cm⁻¹, (358 ± 2) cm⁻¹, (759 ± 2) cm⁻¹, (1199 ± 2) cm⁻¹, (1376 ± 2) cm⁻¹, (1581 ± 2) cm⁻¹ and (1661 ± 2) cm⁻¹; when measured at a temperature in the range of from 20 to 30 °C and a wavelength of 785 nm.
150) A composition comprising the crystalline solvate according to any one of items 140 to 149, which composition is essentially free of any other physical form of tenofovir alafenamide monofumarate and/or hemifumarate.
151) A composition comprising the crystalline solvate according to any one of items 140 to 149, characterized by comprising at most 20 weight% of any other physical form of tenofovir alafenamide monofumarate and/or hemifumarate, based on the weight of the composition.
152) A composition comprising the crystalline solvate according to any one of items 140 to 149, characterized by comprising at most 10 weight% of any other physical form of tenofovir alafenamide monofumarate and/or hemifumarate, based on the weight of the composition.
153) A composition comprising the crystalline solvate according to any one of items 140 to 149, characterized by comprising at most 5 weight% of any other physical form of tenofovir alafenamide monofumarate and/or hemifumarate, based on the weight of the composition.
154) A composition comprising the crystalline solvate according to any one of items 140 to 149, characterized by comprising at most 2 weight% of any other physical form of tenofovir alafenamide monofumarate and/or hemifumarate, based on the weight of the composition.
155) A composition comprising the crystalline solvate according to any one of items 140 to 149, characterized by comprising at most 1 weight% of any other physical form of tenofovir alafenamide monofumarate and/or hemifumarate, based on the weight of the composition.
156) The composition according to any one of items 150 to 155, wherein the any other physical form of tenofovir alafenamide monofumarate is characterized by having a powder X-ray diffractogram comprising reflections at 2-Theta angles of (5.3 ± 0.1)°, (9.8 ± 0.1)°, (10.4 ± 0.1)°, (15.9 ± 0.1)°, (16.2 ± 0.1)° and (16.6 ± 0.1)°, when measured at a temperature in the range of from 20 to 30 °C with Cu-Kalpha_{1,2} radiation having a wavelength of 0.15419 nm.
157) The composition according to any one of items 150 to 155, wherein the any other physical form of tenofovir alafenamide hemifumarate is characterized by having a powder X-ray diffractogram comprising reflections at 2-Theta angles of (6.9 ± 0.2)°, (8.6 ± 0.2)°, (10.0 ± 0.2)°, (11.0 ± 0.2)°, (12.2 ± 0.2)°, (15.9 ± 0.2)°, (16.3 ± 0.2)°, (20.2 ± 0.2)° and (20.8 ± 0.2)°, when measured at a temperature in the range of from 20 to 30 °C with Cu-Kalpha_{1,2} radiation having a wavelength of 0.15419 nm.
158) A process for the preparation of the crystalline solvate of tenofovir alafenamide monofumarate as defined in any one of items 140 to 149 or the composition according to any one of items 150 to 157 comprising:
   (i) providing a solution comprising tenofovir alafenamide monofumarate and a solvent selected from the group consisting of n-propanol, n-butanol and isobutanol;
   (ii) crystallizing the solvate of tenofovir alafenamide monofumarate as defined in any one of items 140 to 149 or the composition as defined in any one of items 150 to 157 from the solution obtained in step (i).
159) The process according to item 158, wherein the tenofovir alafenamide monofumarate concentration of the solution in step (i) is in the range of from 50 to 150 g/L.
160) The process according to item 158, wherein the tenofovir alafenamide monfumarate concentration of the solution in step (i) is in the range of from 75 to 125 g/L.
161) The process according to item 158, wherein the tenofovir alafenamide monofumarate concentration of the solution in step (i) is 100 g/L.
162) The process according to any one of items 158 to 161 further comprising filtering the solution provided in step (i).
163) The process according to any one of items 158 to 162, further comprising seeding the solution provided in step (i) with the crystalline solvate as defined in any one of items 140 to 149.
164) The process according to item 163, wherein the amount of seed crystals is in the range of from 0.1 to 20 weight%, based on the weight of tenofovir alafenamide monofumarate of the solution provided in step (i).
165) The process according to item 163, wherein the amount of seed crystals is in the range of from 1 to 10 weight%, based on the weight of tenofovir alafenamide monofumarate of the solution provided in step (i).
166) The process according to item 163, wherein the amount of seed crystals is in the range of from 1 to 5 weight%, based on the weight of tenofovir alafenamide monofumarate of the solution provided in step (i).
167) The process according to any one of items 158 to 166, wherein the crystallization in step (ii) comprises cooling the solution to a temperature in the range of from 20 to 30 °C or below, and optionally further comprises stirring at the applied temperature for a period in the range of from 2 to 48 hours, preferably from 4 to 24 hours and most preferably from 6 to 18 hours until crystallization is complete.
168) The process according to any one of items 158 to 167, further comprising step (iii) separating at least a part of the crystals obtained in step (ii) from their mother liquor.
169) The process according to item 168, wherein the crystals are separated from their mother liquor by filtration, centrifugation, decantation or solvent evaporation.
170) The process according to item 168, wherein the crystals are separated from their mother liquor by filtration or centrifugation.
171) The process according to item 168, wherein the crystals are separated from their mother liquor by filtration.
172) The process according to any one of items 168 to 171, further comprising step (iv) washing the isolated crystals obtained in step (iii).
173) The process according to item 172, wherein the crystals are washed with an organic solvent and/or water.
174) The process according to item 173, wherein the organic solvent comprises n-propanol, n-butanol or isobutanol.
175) The process according to any one of items 158 to 175 further comprising drying the crystals obtained in any one of steps (iii) or (iv).
176) The process according to item 175, wherein drying is performed at a temperature of 60 °C or less.
177) The process according to item 175, wherein drying is performed at a temperature of 40 °C or less.
178) The process according to item 175, wherein drying is performed at a temperature in the range of from 20 to 30 °C.
179) The process according to any one of items 175 to 178, wherein drying is performed for a period in the range of from 1 to 72 hours.
180) The process according to any one of items 175 to 178, wherein drying is performed for a period in the range of from 2 to 48 hours.
181) The process according to any one of items 175 to 178, wherein drying is performed for a period in the range of from 4 to 24 hours.
182) The process according to any one of items 175 to 178, wherein drying is performed for a period in the range of from 6 to 18 hours.
183) A process for the preparation of tenofovir alafenamide monofumarate form III, wherein the process comprises the step of drying the tenofovir alafenamide monofumarate isobutanol solvate according to item 147.
184) The process according to item 183, wherein drying is performed at reduced pressure.
185) The process according to item 184, wherein the reduced pressure is from 1mbar to 100mbar, such as from 20mbar to 40mbar.
186) The process according to any one of items 183 to 185, wherein drying is performed at a temperature of from 80 to 120 °C.
187) The process according to item 186, wherein drying is performed at a temperature of from 100°C to 115°C.
188) The process according to any one of items 183 to 188, wherein drying is performed for a period of from 30 minutes to 180 minutes.
189) The process according to any one of items 183 to 189, wherein drying is performed for a period of from 50 minutes to 120 minutes.
190) Use of the crystalline form as defined in any one of items 140 to 149 or the composition as defined in any one of items 150 to 157 or as obtained from the process according to any one of items 158 to 182 as seed crystals in a crystallization process for the preparation of the crystalline form as defined in any one of items 140 to 149 or the composition according to any one of items 150 to 157.

### EXAMPLES

### Powder X-ray diffraction

PXRD was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-theta with 40s per step (255 channels) in the angular range of 2° to 40° 2-theta at ambient conditions. A typical precision of the 2-theta values is in the range of ± 0.2° 2-Theta, preferably of ± 0.1° 2-theta. Thus, the diffraction peak of crystalline tenofovir alafenamide monofumarate form II that appears for example at 7.3° 2-Theta can appear in the range of from 7.1 to 7.5° 2-theta, preferably in the range of from 7.2 to 7.4° 2-Theta on most X-ray diffractometers under standard conditions.

### Raman Spectroscopy

Raman spectra were recorded with a RamanRxn 1 Raman spectrometer and a P^{h}AT probe with 6 mm spot size and 250 mm maximum focal length from Kaiser Optical Systems using a 785 nm Invictus laser with 400 mW power with a measurement time of 15 seconds. Spectra were recorded from 1850 to 200 cm⁻¹ with 4 cm⁻¹ resolution at 25 °C and 25% relative humidity. A typical precision of the wavenumber values is in the range of about ± 2 cm⁻¹. Thus, a Raman peak that appears at 1244 cm⁻¹ can appear between 1042 and 1246 cm⁻¹ on most Raman spectrometers under standard conditions. Displayed spectra were baseline corrected with OPUS 7.0 (from Bruker Optik GmbH) using the concave rubberband method with 15 iterations and 64 baseline points.

### Differential scanning calorimetry

Differential scanning calorimetry was performed on a Mettler Polymer DSC R instrument. The sample (4.4 mg form I, 4.2 mg form II and 5.2 mg form S prepared from isobutanol) was heated in a 40 microL aluminum pan with pierced aluminum lid from 25 to 150 °C at a rate of 10 K/min. Nitrogen (purge rate 50 mL/min) was used as purge gas.

### Thermogravimetric analysis

TGA was performed on a Mettler TGA/DSC 1 instrument. The sample (8.8 mg form I, 8.5 mg form II, 7.2 mg form III and 10.1 mg form S prepared from isobutanol) was weighed into a 100 microL aluminum pan closed with an aluminum lid. The lid was automatically pierced at the beginning of the measurement. The sample was heated from 25 to 150 °C at a rate of 10 K/min. Nitrogen (purge rate 50 mL/min) was used as purge gas.

### Gravimetric moisture sorption

GMS was performed with an SPSx-1µ moisture sorption analyzer (ProUmid, Ulm). The measurement cycle was started at ambient relative humidity (RH) of 25%. Relative humidity was then decreased to 5% RH in 5% steps, followed by a further decrease to 3% RH and further to 0% RH. Afterwards, RH was increased from 0% to 95% RH in a sorption cycle in 5% steps and to 0% RH in a desorption cycle in 5% steps. Finally, the RH was increased to ambient relative humidity of 30% in 5% steps.

The time per step was set to a minimum of 2 hours and a maximum of 6 hours. If an equilibrium condition with a constant mass of ± 0.01% within 1 hour was reached before the maximum time for all examined samples the sequential humidity step was applied before the maximum time of 6 hours. If no equilibrium was achieved the consecutive humidity step was applied after the maximum time of 6 hours. The temperature was 25 ± 0.1 °C. Figures 20A and 20B show the equilibrium mass changes (in % on the y-axis) of tenofovir alafenamide monofumarate forms I, II and III during the sorption cycle from 0% to 95% RH measured at a temperature of (25 ± 0.1) °C (on the x-axis). While Figure 20A displays the uncorrected values as obtained from the GMS experiment, Figure 20B shows the corrected values considering the water contents determined by Karl-Fischer titration after the experiment. Coulometric Karl-Fischer titrations were performed on a Metrohm 831 KF Coulometer connected to a KF Thermoprep 832 oven, which was heated to 120 °C for the measurements.

### Example 1: Preparation of crystalline form II of tenofovir alafenamide monofumarate

Tenofovir alafenamide monofumarate form I (2.00 g, prepared according to Reference Example 1 herein below) was suspended in acetonitrile (20 mL) and vigorously stirred at room temperature using a magnetic stirrer. Samples were taken after 6 and 10 days respectively before the crystals were finally collected by filtration after 11 days and dried under vacuum at room temperature for 17 hours.
Yield: 1.71 g (86% of theory)

The two samples and the final product were investigated by powder X-ray diffraction and the results are summarized in table 3:

**Table 3: PXRD results**

| Sample | Isolated after | Result according to PXRD |
|---|---|---|
| A | 6 days | MF form II + traces MF form I |
| B | 10 days | MF form II |
| C | 11 days | MF form II |

The finally isolated form II crystals of tenofovir alafenamide (sample C, isolated after 11 days) were investigated in more detail by means of DSC, TGA and GMS. The results are below discussed in more detail.

### Thermal analysis

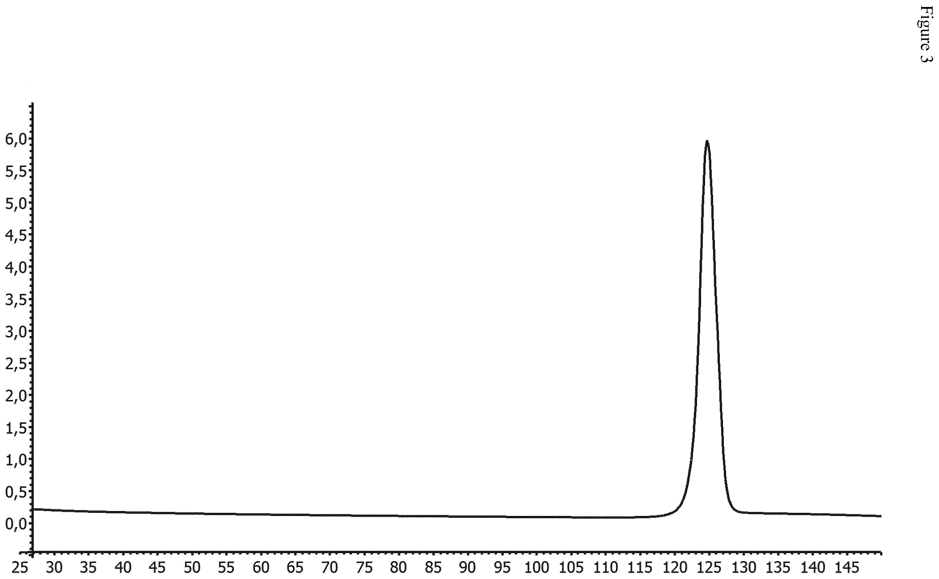

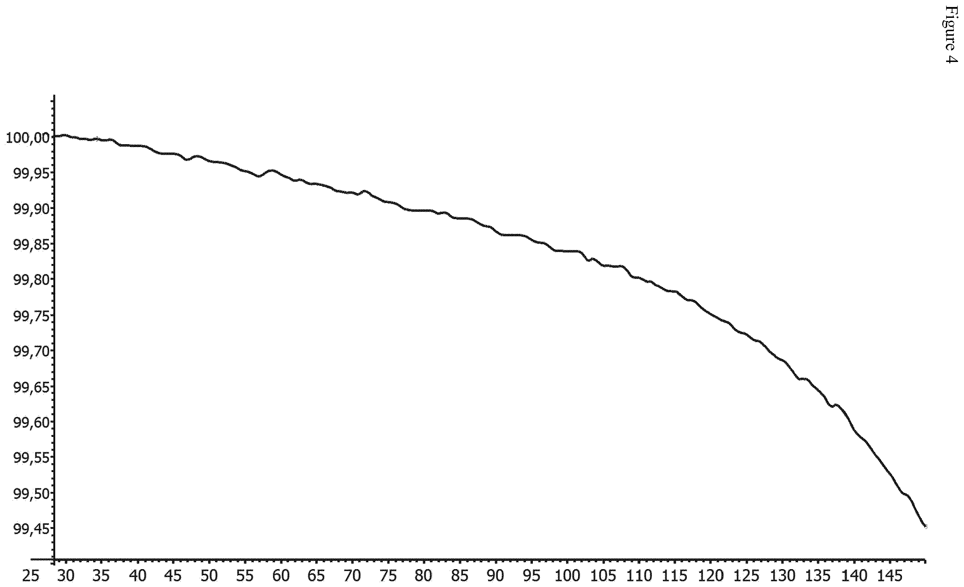

The DSC curve of tenofovir alafenamide monofumarate form II shows a single melting endotherm with an onset temperature of 122.3 °C and a heat of fusion of 104.8 J/g. The DSC curve is displayed in figure 3 herein. Thermogravimetric analysis revealed a mass loss of about 0.3 weight% from the beginning of the measurement up to a temperature of about 130 °C. The TGA curve is displayed in figure 4 herein. From the thermal analysis it can be concluded that form II is an anhydrous and non-solvated form of tenofovir alafenamide monofumarate.

### Gravimetric moisture sorption

Gravimetric moisture sorption of a first sample revealed a weight change of only about 0.4% between 0 and 95% relative humidity, indicating that form II is an anhydrous and non-hygroscopic form of tenofovir alafenamide monofumarate. A GMS experiment of another form II sample prepared in the same manner as described above revealed that the sample showed practically no weight gain during the sorptive mode from 0 to 95% RH. The corresponding GMS isotherm of the latter sample is displayed in Figures 20A and 20B respectively.

### Example 2: Preparation of form III of tenofovir alafenamide monofumarate

Tenofovir alafenamide monofumarate form S (0.5 g, prepared according to example 3 herein below) was stored under vaccum (20-30 mbar) at a temperature of 110 °C for 6 hours, whereupon tenofovir alafenamide monofumarate form III was obtained quantitatively.

The recovered form III crystals of tenofovir alafenamide were investigated in more detail by means of TGA and GMS. The results are discussed below in more detail.

### Thermal Analysis

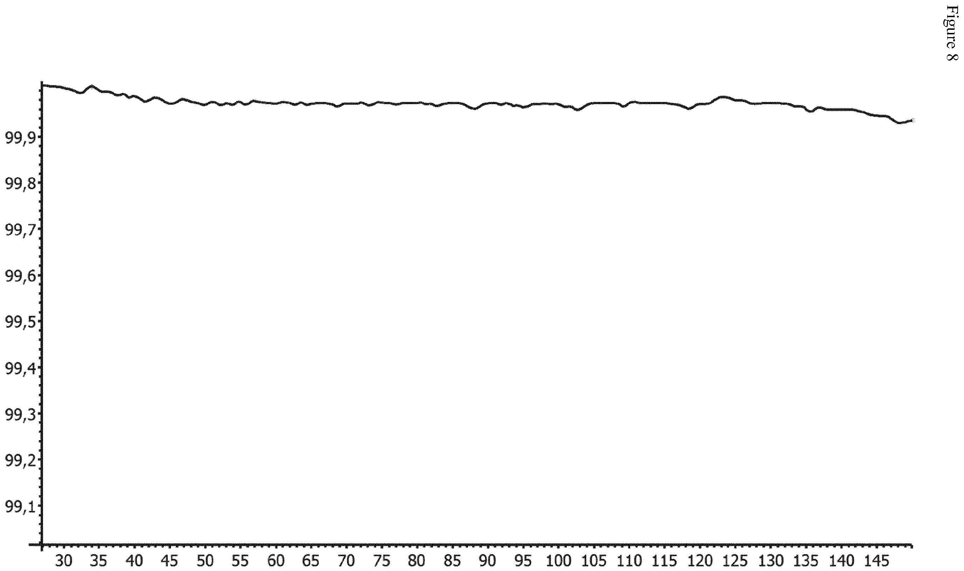

Thermogravimetric analysis revealed practically no mass loss from the beginning of the measurement up to a temperature of about 130 °C. The corresponding TGA curve is displayed in figure 8 herein.

### Gravimetric moisture sorption

A GMS experiment of form III revealed that the sample showed practically no weight gain during the sorptive mode from 0 to 95% RH (see Figures 20A and 20B). Hence, combining the results received from TGA and GMS experiments it can be concluded that form III is an anhydrous, non-solvated and non-hygroscopic form of tenofovir alafenamide monofumarate.

### Example 3: Preparation of form S of tenofovir alafenamide monofumarate from isobutanol

A mixture of tenofovir alafenamide (4.0 g, 8.3 mmol, prepared according to the procedure disclosed in WO 02/08241 A2), fumaric acid (1.0 g, 8.0 mmol) and isobutanol (40 mL) was heated to reflux temperature, whereupon a solution was obtained. The solution was filtered while hot and allowed to cool to room temperature. The obtained mixture was further stirred at room temperature for about 16 hours before the crystals were collected by filtration and air dried for 2 hours to obtain crystalline tenofovir alafenamide form S.
Yield: 4.2 g (81% of theory)

The isolated form S crystals of tenofovir alafenamide were investigated in more detail by means of DSC and TGA. The results are discussed in more detail below.

### Thermal analysis

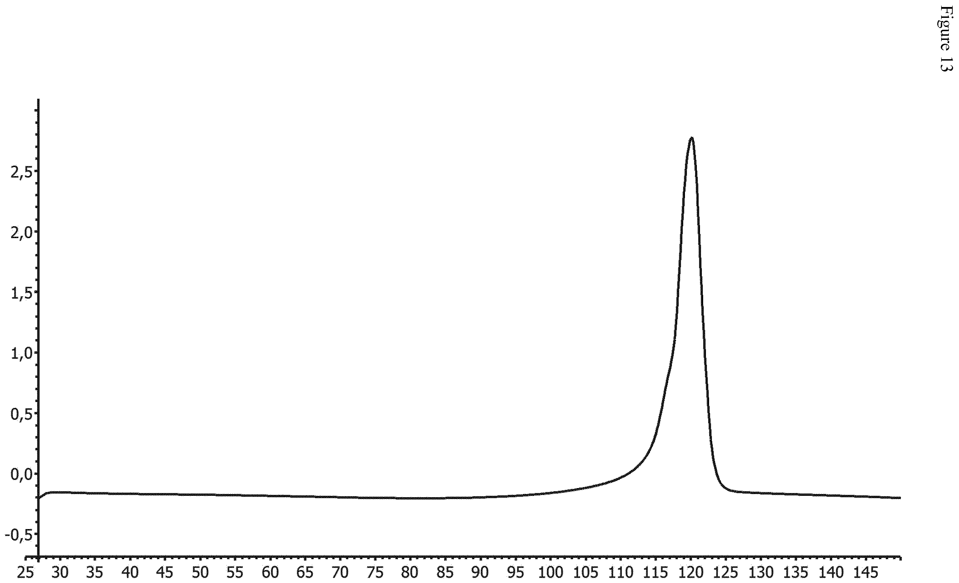

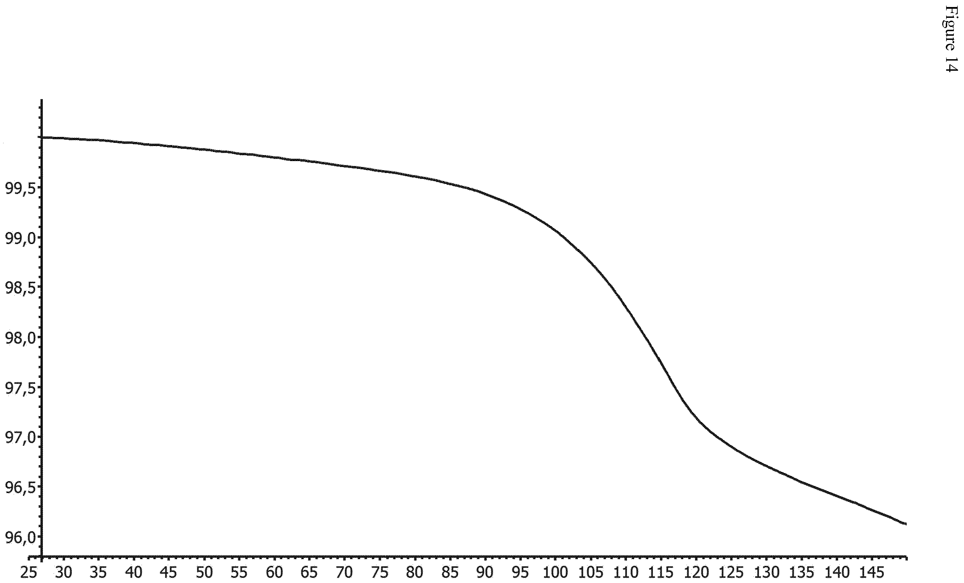

The DSC curve of tenofovir alafenamide monofumarate form S as prepared above shows a single endotherm with an onset temperature of 116.3 °C. The endotherm is caused by a simultaneous melting and desolvation process. The corresponding DSC curve is displayed in figure 13 herein. Thermogravimetric analysis revealed a mass loss of about 3.5 weight% from the beginning of the measurement up to a temperature of about 140 °C. The corresponding TGA curve is displayed in figure 14 herein. From the thermal analysis it can be concluded that the form S is a solvate. The isobutanol solvate obtained according to the above described procedure comprises about 0.3 mol isobutanol per mol tenofovir alafenamide monofumarate.

### Example 4: Preparation of form S of tenofovir alafenamide monofumarate from isobutanol

Tenofovir alafenamide monfumarate (2.0 g, prepared according to the procedure disclosed in WO 02/08241 A2) was dissolved in isobutanol (20 mL) upon heating to reflux temperature. The obtained solution was filtered while hot and allowed to cool to room temperature. The obtained mixture was further stirred at room temperature for about 16 hours before the crystals were collected by filtration and air dried for 2 hours to obtain crystalline tenofovir alafenamide form S.
Yield: 1.7 g (82% of theory)

### Example 5: Preparation of form S of tenofovir alafenamide monofumarate from n-propanol

Tenofovir alafenamide monfumarate (50 mg, prepared according to the procedure disclosed in WO 02/08241 A2) was dissolved in n-propanol (1 mL) upon heating to reflux temperature. The obtained solution was filtered while hot and allowed to cool to room temperature. The obtained mixture was further stirred at room temperature for about 16 hours before the crystals were collected by centrifugation to obtain crystalline tenofovir alafenamide form S.
Yield: 35 mg (68% of theory)

### Example 6: Preparation of form S of tenofovir alafenamide monofumarate from n-butanol

Tenofovir alafenamide monfumarate (50 mg, prepared according to the procedure disclosed in WO 02/08241 A2) was dissolved in n-butanol (1 mL) upon heating to reflux temperature. The obtained solution was filtered while hot and allowed to cool to room temperature. The obtained mixture was further stirred at room temperature for about 16 hours before the crystals were collected by centrifugation to obtain crystalline tenofovir alafenamide form S.
Yield: 35 mg (67% of theory)

### Example 7: Evaluation of stability of tenofovir alafenamide monofumarates

The chemical stability of form I of tenofovir alafenamide monofumarate (prepared as described in reference example 1) was compared with that of form II and form III of tenofovir alafenamide monofumarate (prepared as described herein above). A gradient UPLC determination with external standard was performed according to the following method:

### Solutions:

1. Solvent: buffer, pH = 3.0 : methanol = 400 : 600 (V/V)
2 buffer, pH = 3.0: Weight 0.6 g NaH₂PO₄ into 1 000ml of water and adjust pH with phosphoric(V) acid to pH= 3.0 ± 0.2.
3. buffer, pH = 3.80: Weight 1.5 g CH₃COONH₄ into 1000ml of water and adjust pH with acetic acid to pH= 3.80 ± 0.05.

### Chromatographic conditions:

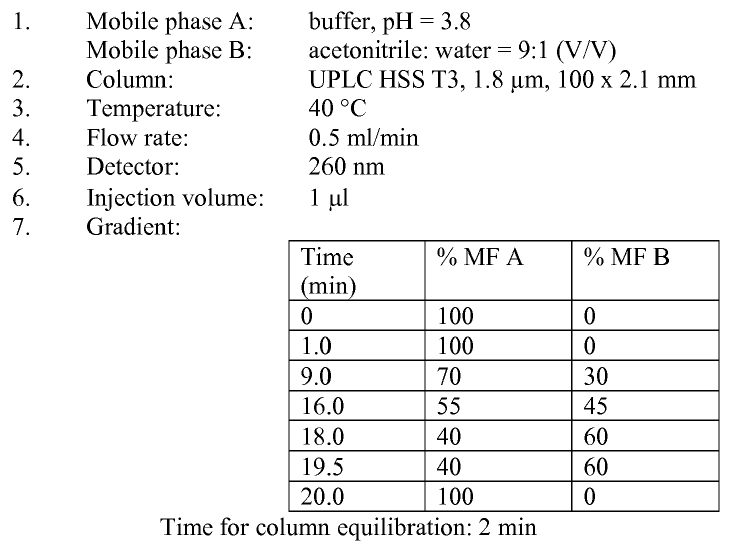

The following stability results were observed:

| | **Increase of total impurities (%)** | | |
|---|---|---|---|
| | **Form I** | **Form II** | **Form III** |
| 60°C 7 days | 0.48 | 0.28 | 0.27 |
| 60°C/30 % RH 7days | 0.41 | 0.48 | 0.29 |
| 60°C/75 % RH 7days | 1.46 | 1.67 | 0.80 |

The samples were exposed to different environmental conditions (60°C closed dish, 60°C/30 % RH, 60°C/75 % RH) for 7 days and the increase of total impurities (in %) was measured by UPLC. Surprisingly, form II and form III of tenofovir alafenamide monofumarate were chemically more stable than form I at 60°C (closed dish). It was also unexpected that among the tested monofumarate forms, tenofovir alafenamide monofumarate form III is the most chemically stable form.

### Example 8

Tenofovir alafenamide free base (24.00 g, prepared according to the procedure disclosed in WO 02/08241 A2) and fumaric acid (6.254 g) were suspended at room temperature in isobutanol (240 mL). The mixture was stirred at room temperature with a magnetic stirrer. After 20 hours n-heptane (280 mL) were added within 10 minutes. After stirring for 1 hour, the precipitated crystals were collected by filtration and sucked dry with the aid of a suction filter. Tenofovir alafenamide monofumarate isobutanol solvate (Form S) was obtained. The obtained tenofovir alafenamide monofumarate isobutanol solvate (Form S) was dried under vacuum (30 mbar) at 110 °C for 90 minutes to provide tenofovir alafenamide monofumarate form III.
Yield: 26.9 g (90% of theory).

### Reference example 1: Preparation of form I of tenofovir alafenamide monofumarate

A mixture of tenofovir alafenamide (1.3 g, 2.7 mmol, prepared according to the procedure disclosed in WO 02/08241 A2), fumaric acid (0.3 g, 2.4 mmol) and acetonitrile (24.6 g) was heated to reflux to dissolve the solids, filtered while hot and cooled to 5 °C for 16 hours. The crystals were collected by filtration, rinsed with acetonitrile (9.6 g) and dried under vacuum at room temperature for 17 hours.
Yield: 1.10 g (85% of theory)

### Thermal analysis

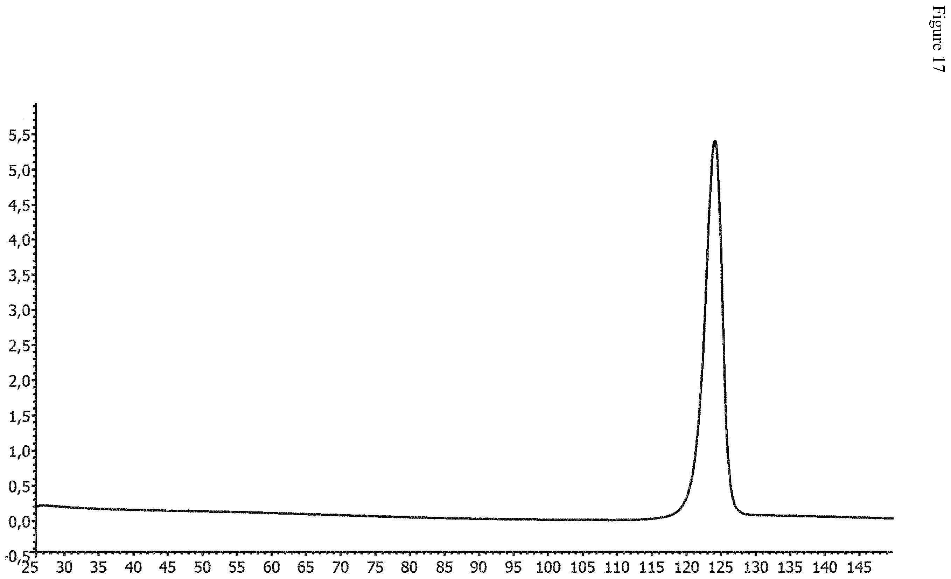

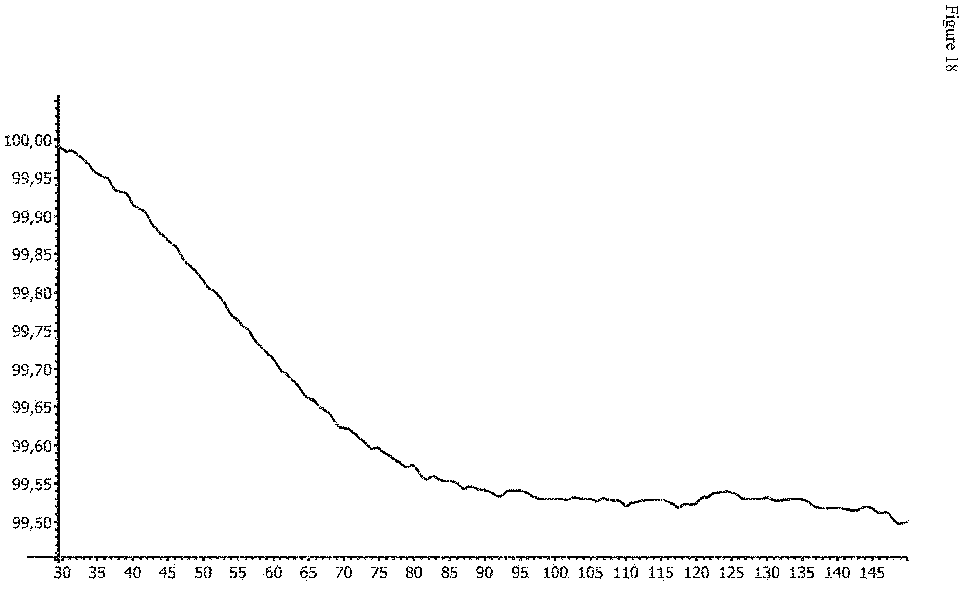

The DSC curve of tenofovir alafenamide monofumarate form I obtained according to reference example 1 herein shows a single melting endotherm with an onset temperature of 121.2 °C and a heat of fusion of 100.9 J/g. The corresponding DSC curve is displayed in figure 17 herein. Thermogravimetric analysis revealed a mass loss of about 0.5 weight% from the beginning of the measurement up to a temperature of about 130 °C. The corresponding TGA curve is displayed in figure 18 herein.

### Gravimetric moisture sorption

Gravimetric moisture sorption revealed a weight change of about 1.5% between 0 and 95% relative humidity, indicating that form I is an anhydrous and slightly hygroscopic form of tenofovir alafenamide monofumarate. A GMS experiment of another form I sample prepared in the same manner as described above revealed a weight gain of 1.1% during the sorptive mode from 0 to 95% RH confirming the hygroscopic behavior of this form. The corresponding GMS isotherm of the latter sample is displayed in Figures 20A and 20B respectively.

### Reference example 2: Preparation of tenofovir alafenamide monofumarate according to example 19 in combination with example 13 of WO 2015/107451 A1

A mixture of tenofovir alafenamide (2.0 g, 4.2 mmol, prepared according to the procedure disclosed in WO 02/08241 A2), benzoic acid (0.5 g, 4.1 mmol) and acetone (5 mL) was heated to 55 °C and stirred at this temperature until the solid was completely dissolved. The solution was then slowly cooled to 22 °C. The obtained solid was collected by filtration, washed with acetone and dried at room temperature under vacuum (20 mbar). Subsequently, the solid was dissolved in methylene chloride (3 mL) and water (1.5 mL) before the pH was adjusted to 4-4.5 with a sodium bicarbonate solution. The reaction mass was stirred and the organic and aqueous layers were separated. The organic layer was washed with water (1.5 mL), stirred and the layers were again separated. The organic layer was then dried with sodium sulphate and the methylene chloride was completely removed under vacuum. The obtained residue was dissolved in ethanol (1.8 mL) before fumaric acid (88 mg) was added. Then, the mixture was heated to 65 °C followed by cooling to 5 °C. The thus obtained solid was collected by filtration, washed with chilled ethanol and dried at room temperature under vacuum (20 mbar). PXRD confirmed the receipt of tenofovir alafenamide monofumarate form I.

### Reference example 3: Preparation of tenofovir alafenamide monofumarate according to example 20 of WO 2015/107451 A1

A mixture of tenofovir alafenamide (2.0 g, 4.2 mmol, prepared according to the procedure disclosed in WO 02/08241 A2), fumaric acid (0.5 g, 4.1 mmol) and methanol (10 mL) was heated to reflux and stirred at this temperature until the solid was completely dissolved. The solution was slowly cooled to 22 °C and further cooled to 0 °C. The obtained solid was collected by filtration, washed with acetonitrile and sucked dry on the filter. PXRD confirmed the receipt of tenofovir alafenamide monofumarate form I.

### Reference example 4: Preparation of tenofovir alafenamide monofumarate according to example 1 of CN 105237571 A

A mixture of tenofovir alafenamide (952 mg, 2.0 mmol, prepared according to the procedure disclosed in WO 02/08241 A2), fumaric acid (232 mg, 2.0 mmol) and acetonitrile (10 mL) was heated to reflux and stirred at this temperature until the solid was completely dissolved. The solution was filtered while hot before it was cooled to 22 °C and further stirred at this temperature overnight. The obtained solid was collected by filtration, washed with acetonitrile and dried at room temperature under vacuum (20 mbar) to obtain 1020 mg of tenofovir alafenamide monofumarate. PXRD confirmed the receipt of a mixture of tenofovir alafenamide monofumarate form I and tenofovir alafenamide hemifumarate.

## Claims

1. A crystalline form of tenofovir alafenamide monofumarate (form II), **characterized by** having a powder X-ray diffractogram comprising reflections at 2-Theta angles of (7.3 ± 0.2)°, (9.4 ± 0.1)° and (10.1 ± 0.1)°, when measured at a temperature in the range of from 20 to 30 °C with Cu-Kalpha_{1,2} radiation having a wavelength of 0.15419 nm.

2. The crystalline form of claim 1, wherein the powder X-ray diffractogram comprises an additional reflection at one or more 2-Theta angle(s) selected from the group consisting of (5.6 ± 0.1)°, (13.0 ± 0.1)° and (28.4 ± 0.1)°.

3. Process for the preparation of the crystalline form according to claim 1 or 2 comprising the steps of:
(i) providing tenofovir alafenamide monofumarate in solid form;
(ii) slurrying tenofovir alafenamide monofumarate provided in step (i) in a solvent comprising acetonitrile for at least 10 days.

4. A crystalline form of tenofovir alafenamide monofumarate (form III), **characterized by** having a powder X-ray diffractogram comprising reflections at 2-Theta angles of (5.6 ± 0.1)°, (7.3 ± 0.2)° and (10.5 ± 0.1)°, when measured at a temperature in the range of from 20 to 30 °C with Cu-Kalpha_{1,2} radiation having a wavelength of 0.15419 nm.

5. The crystalline form of claim 4, wherein the powder X-ray diffractogram comprises an additional reflection at one or more 2-Theta angle(s) selected from the group consisting of (9.4 ± 0.1)°, (12.6 ± 0.1)° and (17.0 ± 0.1)°.

6. Process for the preparation of the crystalline form according to claim 4 or 5 comprising:
(i) providing a crystalline solvate of tenofovir alafenamide monofumarate as defined in claim 7 or 8 (form S);
(ii) at least partially desolvating the crystalline solvate provided in step (i) at a temperature in the range of from 100 to 115 °C;

7. A crystalline solvate of tenofovir alafenamide monofumarate (form S), **characterized by** having a powder X-ray diffractogram comprising reflections at 2-Theta angles of (4.8 ± 0.2)°, (10.3 ± 0.2)° and (10.6 ± 0.2)°, when measured at a temperature in the range of from 20 to 30 °C with Cu-Kalpha_{1,2} radiation having a wavelength of 0.15419 nm.

8. The crystalline solvate of claim 7, wherein the powder X-ray diffractogram comprises an additional reflection at one or more 2-Theta angle(s) selected from the group consisting of (15.1 ± 0.2)°, (15.3 ± 0.2)° and (24.0 ± 0.2)°.

9. Process for the preparation of the crystalline solvate according to claim 7 or 8 comprising:
(i) providing a solution comprising tenofovir alafenamide monofumarate and a solvent selected from the group consisting of n-propanol, n-butanol and isobutanol;
(ii) crystallizing tenofovir alafenamide monofumarate form S as defined in claims 7 or 8 from the solution obtained in step (i).

10. A composition comprising one or more of the crystalline forms as defined in claims 1 or 2, 4 or 5 and 7 or 8 and less than 20 weight%, 10 weight%, 5 weight% or 1 weight% of any other physical form of tenofovir alafenamide monofumarate and/or hemifumarate, based on the weight of the composition.

11. The composition according to claim 10, wherein the any other physical form of tenofovir alafenamide monofumarate is **characterized by** having a powder X-ray diffractogram comprising reflections at 2-Theta angles of (5.3 ± 0.1)°, (9.8 ± 0.1)°, (10.4 ± 0.1)°, (15.9 ± 0.1)°, (16.2 ± 0.1)° and (16.6 ± 0.1)°, when measured at a temperature in the range of from 20 to 30 °C with Cu-Kalpha_{1,2} radiation having a wavelength of 0.15419 nm.

12. The composition according to claim 10, wherein the any other physical form of tenofovir alafenamide hemifumarate is **characterized by** having a powder X-ray diffractogram comprising reflections at 2-Theta angles of (6.9 ± 0.2)°, (8.6 ± 0.2)°, (10.0 ± 0.2)°, (11.0 ± 0.2)°, (12.2 ± 0.2)°, (15.9 ± 0.2)°, (16.3 ± 0.2)°, (20.2 ± 0.2)° and (20.8 ± 0.2)°, when measured at a temperature in the range of from 20 to 30 °C with Cu-Kalpha_{1,2} radiation having a wavelength of 0.15419 nm.

13. A pharmaceutical composition comprising an effective amount of the crystalline form as defined in any one of claims 1 or 2 and/or 4 or 5 and one or more pharmaceutically acceptable excipient(s).

14. The pharmaceutical composition of claim 13 for use as a medicament.

15. The pharmaceutical composition of claim 13 for use in the treatment and/or prophylaxis of viral infections, optionally viral infections caused by DNA viruses, RNA viruses, herpesviruses, retroviruses, hepadnaviruses, papillomavirus, hantavirus, adenoviruses and HIV.

## Patentansprüche

1. Kristalline Form von Tenofovir-Alafenamld-Monofumarat (Form II), **dadurch gekennzeichnet, dass** sie ein Pulver-Röntgendiffraktogramm aufweist, das Reflexe bei 2-Theta-Winkeln von (7,3 ± 0,2)°, (9,4 ± 0,1)° und (10,1 ± 0,1)° umfasst, wenn bei einer Temperatur in dem Bereich von 20 bis 30 °C mit Cu-Kalpha_{1,2}-Strahlung mit einer Wellenlänge von 0,15419 nm gemessen wird.

2. Kristalline Form nach Anspruch 1, wobei das Pulver-Röntgendiffraktogramm einen zusätzlichen Reflex bei einem oder mehreren 2-Theta-Winkel(n), die aus der Gruppe bestehend aus (5,6 ± 0,1)°, (13,0 ± 0,1)° und (28,4 ± 0,1)° ausgewählt sind, umfasst.

3. Verfahren zur Herstellung der kristallinen Form nach Anspruch 1 oder 2, umfassend die folgenden Schritte:
(i) Bereitstellen von Tenofovir-Alafenamid-Monofumarat in fester Form;
(ii) Aufschlämmen des in Schritt (i) bereitgestellten Tenofovir-Alafenamid-Monofumarats in einem Lösungsmittel umfassend Acetonitril für mindestens 10 Tage.

4. Kristalline Form von Tenofovir-Alafenamid-Monofumarat (Form III), **dadurch gekennzeichnet, dass** sie ein Pulver-Röntgendiffraktogramm aufweist, das Reflexe bei 2-Theta Winkeln von (5,6 ± 0,1)°, (7,3 ± 0,2)° und (10,5 ± 0,1)° umfasst, wenn bei einer Temperatur in dem Bereich von 20 bis 30 °C mit Cu-Kalpha_{1,2}-Strahlung mit einer Wellenlänge von 0,15419 nm gemessen wird.

5. Kristalline Form nach Anspruch 4, wobei das Pulver-Röntgendiffraktogramm einen zusätzlichen Reflex bei einem oder mehreren 2-Theta-Winkel(n), die aus der Gruppe bestehend aus (9,4 ± 0,1)°, (12,6 ± 0,1)° und (17,0 ± 0,1)° ausgewählt sind, umfasst.

6. Verfahren zur Herstellung der kristallinen Form nach Anspruch 4 oder 5, umfassend:
(i) Bereitstellung eines kristallinen Solvats von Tenofovir-Alafenamid-Monofumarat nach Anspruch 7 oder 8 (Form S);
(ii) zumindest teilweises Desolvatlsieren des in Schritt (l) bereitgestellten kristallinen Solvats bei einer Temperatur im Bereich von 100 bis 115°C;

7. Kristallines Solvat von Tenofovir-Alafenamid-Monofumarat (Form S), **dadurch gekennzeichnet, dass** es ein Pulver-Röntgeridiffraktogramm aufweist, das Reflexe bei 2-Theta-Winkeln von (4,8 ± 0,2)°, (10,3 ± 0,2)° und (10,6 ± 0,2)° umfasst, wenn bei einer Temperatur im Bereich von 20 bis 30 °C mit Cu-Kalpha_{1,2}-Strahlung mit einer Wellenlänge von 0,15419 nm gemessen wird.

8. Kristallines Solvat nach Anspruch 7, wobei das Pulver-Röntgendiffraktogramm einen zusätzlichen Reflex bei einem oder mehreren 2-Theta-Winkel(n), die aus der Gruppe bestehend aus (15,1 ± 0,2)°, (15,3 ± 0,2)° und (24,0 ± 0,2)° ausgewählt sind, umfasst.

9. Verfahren zur Herstellung des kristallinen Solvats nach Anspruch 7 oder 8, umfassend:
(i) Bereitstellung einer Lösung, die Tenofovir-Alafenamid-Monofumarat und ein Lösungsmittel, ausgewählt aus der Gruppe bestehend aus *n*-Propanol, *n-*Butanol und Isobutanol, umfasst;
(ii) Kristallisieren von Tenofovir-Alafenamid-Monofumarat Form S wie in den Ansprüchen 7 oder 8 definiert aus der in Schritt (i) erhaltenen Lösung.

10. Zusammensetzung, die eine oder mehrere der kristallinen Formen wie in den Ansprüchen 1 oder 2, 4 oder 5 und 7 oder 8 definiert, und weniger als 20 Gew.-%, 10 Gew.-%, 5 Gew.-% oder 1 Gew.-% einer anderen physikalischen Form von Tenofovir-Alafenamid-Monofumarat und/oder Hemifumarat, umfasst, bezogen auf das Gewicht der Zusammensetzung.

11. Zusammensetzung nach Anspruch 10, wobei die jegliche andere physikalische Form von Tenofovir-Alafenamid-Monofumarat **dadurch gekennzeichnet ist, dass** sie ein Pulver-Röntgendiffraktogramm mit Reflexen bei 2-Theta-Winkeln von (5,3 ± 0,1)°, (9,8 ± 0,1)°, (10,4 ± 0,1)°. (15,9 ± 0,1)°, (16,2 ± 0,1)° und (16,6 ± 0,1)°, wenn bei einer Temperatur im Bereich von 20 bis 30 °C mit Cu-Kalpha_{1,2}-Strahlung mit einer Wellenlänge von 0,15419 nm gemessen wird, umfasst.

12. Zusammensetzung nach Anspruch 10, wobei die jegliche andere physikalische Form von Tenofovir-Alafenamid-Hemifumarat **dadurch gekennzeichnet ist, dass** sie ein Pulver- Röntgendlffraktogramm mit Reflexnen bei 2-Theta-Winkeln von (6,9 ± 0,2)°, (8,6 ± 0,2)°, (10,0 ± 0,2)°, (11,0 ± 0,2)°, (12,2 ± 0,2)°, (15,9 ± 0,2)°, (16,3 ± 0,2)°, (20,2 ± 0,2)° und (20,8 ± 0,2)°, wenn bei einer Temperatur im Bereich von 20 bis 30 °C mit Cu-Kalpha_{1,2}-Strahlung mit einer Wellenlänge von 0,15419 nm gemessen wird, umfasst.

13. Pharmazeutische Zusammensetzung, umfassend eine wirksame Menge der kristallinen Form, wie in irgendeinem der Ansprüche 1 oder 2 und/oder 4 oder 5 definiert, und einen oder mehrere pharmazeutisch akzeptable(n) Hilfsstoff(e).

14. Pharmazeutische Zusammensetzung nach Anspruch 13 zur Verwendung als Medikament.

15. Pharmazeutische Zusammensetzung nach Anspruch 13 zur Verwendung bei der Behandlung und/oder Prophylaxe von Virusinfektionen, optional Virusinfektionen verursacht durch DNA-Viren, RNA-Viren, Herpesviren, Retroviren, Hepadnaviren, Papillomaviren, Hantaviren, Adenoviren und HIV.

## Revendications

1. Forme cristalline de monofumarate de ténofovir alafénamide (forme II), **caractérisée en ce qu'**elle a un diagramme de diffraction des rayons X sur poudre comprenant des réflexions aux angles 2-théta de (7,3 ± 0,2)°, (9,4 ± 0,1)° et (10,1 ± 0,1)°, lorsque la mesure est effectuée à une température dans la plage de 20 à 30 °C avec un rayonnement Cu-Kalpha_{1,2} ayant une longueur d'onde de 0,15419 nm.

2. Forme cristalline selon la revendication 1, dans laquelle le diagramme de diffraction des rayons X sur poudre comprend une réflexion supplémentaire à un ou plusieurs angles 2-théta sélectionnés dans le groupe consistant en (5,6 ± 0,1)°, (13,0 ± 0,1)° et (28,4 ± 0,1)°.

3. Procédé de préparation de la forme cristalline selon la revendication 1 ou 2 comprenant les étapes suivantes :
(i) fourniture de monofumarate de ténofovir alafénamide sous forme solide ;
(ii) mise en suspension du monofumarate de ténofovir alafénamide fourni à l'étape (i) dans un solvant comprenant l'acétonitrile pendant au moins 10 jours.

4. Forme cristalline de monofumarate de ténofovir alafénamide (forme III), **caractérisée en ce qu'**elle a un diagramme de diffraction des rayons X sur poudre comprenant des réflexions aux angles 2-théta de (5,6 ± 0,1)°, (7,3±0,2)° et (10,5 ± 0,1)°, lorsque la mesure est effectuée à une température dans la plage de 20 à 30 °C avec un rayonnement Cu-Kalpha^{1,2} ayant une longueur d'onde de 0,15419 nm.

5. Forme cristalline selon la revendication 4, dans laquelle le diagramme de diffraction des rayons X sur poudre comprend une réflexion supplémentaire à un ou plusieurs angles 2-théta sélectionnés dans le groupe consistant en (9,4 ± 0,1)°, (12,6 ± 0,1)° et (17,0 ± 0,1)°.

6. Procédé de préparation de la forme cristalline selon la revendication 4 ou 5 comprenant :
(i) la fourniture d'un solvate cristallin de monofumarate de ténofovir alafénamide selon la revendication 7 ou 8 (forme S) ;
(ii) la désolvatation au moins partielle du solvate cristallin fourni à l'étape (i) à une température dans la plage de 100 à 115 °C.

7. Solvate cristallin de monofumarate de ténofovir alafénamide (forme S), **caractérisé en ce qu'**il a un diagramme de diffraction des rayons X sur poudre comprenant des réflexions aux angles 2-théta de (4,8 ± 0,2)°, (10,3 ± 0,2)° et (10,6 ± 0,2)°, lorsque la mesure est effectuée à une température dans la plage de 20 à 30 °C avec un rayonnement Cu-Kalpha^{1,2} ayant une longueur d'onde de 0,15419 nm.

8. Solvate cristallin selon la revendication 7, dans lequel le diagramme de diffraction des rayons X sur poudre comprend une réflexion supplémentaire à un ou plusieurs angles 2-théta sélectionnés dans le groupe consistant en (15,1 ± 0,2)°, (15,3 ± 0,2)° et (24,0 ± 0,2)°,

9. Procédé de préparation du solvate cristallin selon la revendication 7 ou 8 comprenant :
(i) la fourniture d'une solution comprenant le monofumarate de ténofovir alafénamide et un solvant sélectionné dans le groupe consistant en le *n-*propanol, le *n*-butanol et l'isobutanol ;
(ii) la cristallisation de la forme S de monofumarate de ténofovir alafénamide selon les revendications 7 ou 8 à partir de la solution obtenue à l'étape (i).

10. Composition comprenant une ou plusieurs des formes cristallines selon les revendications 1 ou 2, 4 ou 5 et 7 ou 8 et moins de 20 % en poids, 10 % en poids, 5 % en poids ou 1 % en poids d'une autre forme physique quelconque de monofumarate et/ou hémifumarate de ténofovir alafénamide, sur la base du poids de la composition.

11. Composition selon la revendication 10, dans laquelle l'autre forme physique quelconque de monofumarate de ténofovir alafénamide est **caractérisée en ce qu'**elle a un diagramme de diffraction des rayons X sur poudre comprenant des réflexions aux angles 2-théta de (5,3 ± 0,1)°, (9,8 ±0,1)°, (10,4±0,1)°, (15,9 ± 0,1)°, (16,2 ± 0,1)° et (16,6 ± 0,1)°, lorsque la mesure est effectuée à une température dans la plage de 20 à 30 °C avec un rayonnement Cu-Kalpha^{1,2} ayant une longueur d'onde de 0,15419 nm.

12. Composition selon la revendication 10, dans laquelle l'autre forme physique quelconque d'hémifumarate de ténofovir alafénamide est **caractérisée en ce qu'**elle a un diagramme de diffraction des rayons X sur poudre comprenant des réflexions aux angles 2-théta de (8,9 ± 0,2)°, (8,6 ± 0,2)°, (10,0 ± 0,2)°, (11,0 ± 0,2)°, (12,2 ± 0,2)°, (15,9 ± 0,2)°, (16,3 ± 0,2)", (20,2 ± 0,2)° et (20,8 ± 0,2)°, lorsque la mesure est effectuée à une température dans la plage de 20 à 30 °C avec un rayonnement Cu-Kalpha^{1,2} ayant une longueur d'onde de 0,15419 nm.

13. Composition pharmaceutique comprenant une quantité efficace de la forme cristalline selon l'une quelconque des revendications 1 ou 2 et/ou 4 ou 5 et un ou plusieurs excipients pharmaceutiquement acceptables.

14. Composition pharmaceutique selon la revendication 13 pour son utilisation en tant que médicament.

15. Composition pharmaceutique selon la revendication 13 pour son utilisation dans le traitement et/ou la prophylaxie d'infections virales, facultativement d'infections virales provoquées par des virus à ADN, des virus à ARN, des herpesvirus, des rétrovirus, des hepadnavirus, un papillomavirus, un hantavirus, des adénovirus et le VIH.
